(19) Europäisches Patentamt
European Patent Office
Office européen des brevets

(11) Publication number: **0 103 895 A2**

(12) # EUROPEAN PATENT APPLICATION

(21) Application number: 83109398.4

(22) Date of filing: 21.09.83

(51) Int. Cl.³: C 07 C 83/00
A 61 K 31/13, A 61 K 31/16

(30) Priority: 22.09.82 JP 165268/82
26.03.83 JP 50768/83
26.03.83 JP 50769/83
26.03.83 JP 50770/83

(43) Date of publication of application:
28.03.84 Bulletin 84/13

(84) Designated Contracting States:
BE CH DE GB IT LI LU NL

(71) Applicant: MEIJI SEIKA KABUSHIKI KAISHA
4-16 Kyobashi 2-chome
Chuo-ku, Tokyo-To(JP)

(72) Inventor: Hirayama, Masao
5-33-17, Shonan Takatori
Yokosuka-Shi Kanagawa-Ken(JP)

(72) Inventor: Oya, Zenichiro
1-24-24-205, Kaminomiya Tsurumi-Ku
Yokohama-Shi Kanagawa-Ken(JP)

(72) Inventor: Hirano, Fumiya
3-520, Tenno-Cho Danchi 2-24-2, Tenno-Cho
Hodogaya-Ku Yokohama-Shi Kanagawa-Ken(JP)

(72) Inventor: Fukatsu, Shunzo
1-13, Ichigaya Tamachi
Shinjuku-Ku Tokyo-To(JP)

(72) Inventor: Watanabe, Tetsuro
390-3, Matsumi-Cho 2-Chome Kanagawa-Ku
Yokohama-Shi Kanagawa-Ken(JP)

(72) Inventor: Takeda, Ueto
1-31-1-109, Oomori Higashi
Oota-Ku Tokyo-To(JP)

(74) Representative: Dr. Elisabeth Jung Dr. Jürgen Schirdewahn Dr. Gerhard Schmitt-Nilson Dr. Gerhard B. Hagen Dipl.-Ing. Peter Hirsch
Clemensstrasse 30 Postfach 40 14 68
D-8000 München 40(DE)

(54) **Novel hydroxylamine derivatives, production and use thereof.**

(57) Novel hydroxylamine derivatives, uses thereof in anthelmintic methods and compositions, and methods of production thereof are disclosed. The novel compounds are represented by the formula (A):

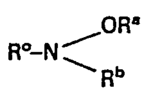

(A)

where: R° is hydrogen atom or a group of $R^1$-CO- where $R^1$ is hydrogen atom, a lower alkyl, a lower alkenyl, phenyl or a substituted phenyl; $R^a$ is a lower alkyl, a lower alkenyl, or a lower alkynyl; and $R^b$ is a lower alkenyl or a lower alkynyl, with a proviso that some of the combinations of R°, $R^a$ and $R^b$ are excluded. The uses and the methods of production are directed to hydroxylamine derivatives of a broader scope.

EP 0 103 895 A2

# NOVEL HYDROXYLAMINE DERIVATIVES, PRODUCTION AND USE THEREOF

## BACKGROUND OF THE INVENTION

### Field of the art

The present invention relates to novel hydroxylamine derivatives each having an unsaturated hydrocarbyl group, production of these compounds and use of these compounds, namely, their use in anthelmintic processes.

### Prior art

Some hydroxylamine derivatives each having an unsaturated hydrocarbyl group are known in the art, namely, N-propargyl-N-benzoyl-O-methylhydroxylamine in Moore et al: J. Medical Chemistry, Vol. 12, p.45 (1969); N-allyl-N-benzoyl-O-propylhydroxylamine in Johnson et al: J. Organic Chemistry, Vol. 36, p.284 (1971); O-methyl-N-allylhydroxylamine in French Patent No.2,122,338; and O,N-diallylhydroxylamine in Eur. Patent Application No. 29,171 (1981).

To the best of our knowledge, it has not been known in the art heretofore that hydroxylamine derivatives each having an unsaturated hydrocarbyl group such as those referred to above have anthelmintic activity for animals.

## SUMMARY OF THE INVENTION

The hydroxylamine derivatives in accordance with the present invention are each represented by the formula

A:

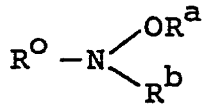

(A)

where: $R^o$ is a hydrogen atom or a group of the formula $R^1$-CO- where $R^1$ is a hydrogen atom or an alkyl or alkenyl having up to 5 carbon atoms, phenyl, or a substituted phenyl, the substituent being an alkyl having up to 3 carbon atoms, a halogen atom or nitro,

$R^a$ is an alkyl, alkenyl or alkynyl having up to 5 carbon atoms; and

$R^b$ is an alkenyl or alkynyl having up to 5 carbon atoms,

the combination of $R^o$, $R^a$ and $R^b$ being selected from the group consisting of:

(1) the combination in which

$R^o$ is $R^1$-CO-, $R^1$ being defined hereinabove,

$R^a$ is an alkyl or alkenyl having up to 5 carbon atoms, and

$R^b$ is $-CH_2C{\equiv}CH$,

provided that $R^a$ is not an alkyl when $R^1$ is phenyl;

(2) the combination in which

$R^o$ is $R^1$-CO-, $R^1$ being as defined hereinabove,

$R^a$ is an alkyl having up to 5 carbon atoms, and

$R^b$ is $-CH_2CH{=}CH{-}R^3$, $R^3$ being hydrogen or methyl,

provided that $R^a$ is not an alkyl having 3 carbon

atoms when $R^1$ is phenyl;

(3) -

(4) the combination in which

$R^o$ is H,

$R^a$ is as defined hereinabove, and

$R^b$ is as defined hereinabove,

provided that $R^a$ is not methyl, or $R^a$ and $R^b$ are not allyl simultaneously; and

(5) the combination in which

$R^o$ is $R^1$-CO-, $R^1$ being as defined hereinabove,

$R^a$ is $-CH_2C\equiv CH$, and

$R^b$ is $-CH_2C\equiv CH$.

Preferred combounds of formula A are those in which $R^1$ is an alkyl having up to 5 carbon atoms. Examples of these compounds are as follows:

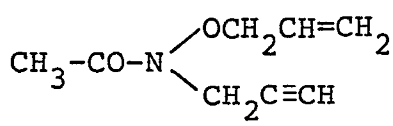,

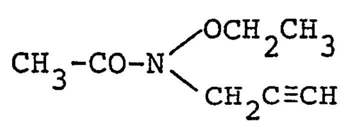,

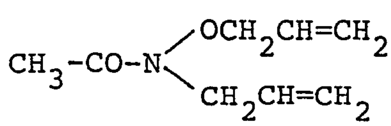, and

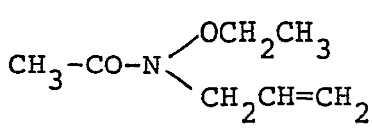.

Other preferred compounds of formula A are those

in which $R^o$ is hydrogen and which are each in the form of a salt with an acid. An example of these compounds is:

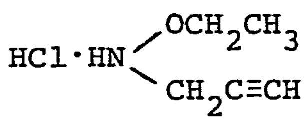

The compounds represented by formula A have been obtained sequentially, namely, the compounds of a formula:

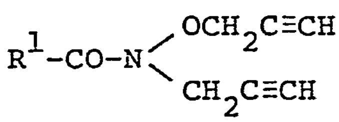

where $R^1$ is defined hereinabove, and then the remainder.

In another aspect thereof, the present invention relates to an anthelmintic method and to an anthelmintic composition wherein an anthelmintic activity of the compounds represented by the following formula A' is utilized.

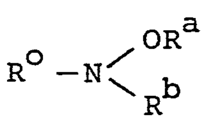

(A')

wherein $R^o$, $R^a$ and $R^b$ are the same as those defined in the formula A except for the following two points.

(1) Combinations of $R^o$, $R^a$ and $R^b$ are selected from the group of (1), (2), (4) and (5) in the formula A, whereas the combinations are selected from the group of (1) through (5) inclusive in the formula A'.

The group of (3) is:

(3) the combination in which
$R^o$ is $R^1$-CO-, $R^1$ being defined hereinabove,
$R^a$ is $-CH_2CH=CH-R^3$, and
$R^b$ is $-CH_2CH=CH-R^4$,
$R^3$ and $R^4$ being hydrogen or methyl, respectively.

For the group of (3), see claim 17.

(2) Combinations of $R^o$, $R^a$ and $R^b$ include provisos in the formula A, whereas they do not in the formula A'.

In view of the relationship between the formula (A) and (A') as shown above, the description given hereinbelow of the compounds of the formula A applies also to the compounds of the formula (A') except for the difference due to the relationship shown above. Accordingly, the present specification is to be read in this way.

The compounds of the formula A' are of low toxicity to warm-blooded animals and have high anthelmintic activity. The use of the compounds thus provides convenient and effective anthelmintic method and composition. The anthelmintic method comprises administering orally to an animal afflicted with a parasite a compound represented by the formula (A'). The anthelmintic composition comprises a compound represented by the formula (A') and a carrier.

The present invention, in still another aspect thereof, provides methods of producing compounds represented by the formula (A'). The methods depend on the type of the compound to be produced. Details of the methods will be given hereinlater.

## DETAILED DESCRIPTION OF THE INVENTION

### Compounds and production thereof

Hydroxylamine derivatives containing unsaturated hydrocarbyl groups of this invention can be divided into

five main types of compounds (I-1 to 5).

The following description of substituted groups in compounds are commonly applicable to the compounds concerned provided that contradictions do not arise mutually therebetween.

<u>Compounds (I-1): Definition</u>

Compound (I-1) are N-propargyl-N-acylhydroxylamine derivatives as is represented by the following formula.

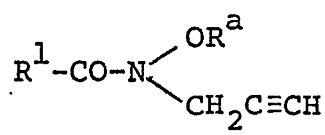 (I-1)

[wherein $R^1$ and $R^a$ are the same as those presented in the combination (1) of $R^o$, $R^a$, and $R^b$ in the definition of the compounds of the formula (A).]

In formula (I-1), examples of $R^1$ which constitutes an N-acyl group preferably include: (1) hydrogen, and linear or branched lower alkyl groups having about 1 to 5 carbon atoms such as methyl, ethyl, n-propyl, n-butyl, isobutyl, and n-pentyl; (2) linear or branched lower alkenyl groups having about 3 to 5 carbon atoms such as 2-propenyl, 2-butenyl, and isobutenyl; (3) middle alkyl or alkenyl groups having about 6 to 8 carbon atoms; (4) higher alkyl or alkenyl groups such as the alkyl chain of lauric acid ($C_{11}H_{23}$), the alkyl chain of stearic acid ($C_{17}H_{35}$), and the alkenyl chain of oleic acid ($C_{17}H_{31}$); (5) phenyl groups; and (6) phenyl groups substituted by halogen atoms such as fluorine, chlorine, bromine,

iodine and the like, phenyl groups substituted by lower alkyl groups such as methyl and ethyl having 3 or less carbon atoms, phenyl groups substituted by nitro groups, and the like. Preferable phenyl groups are those substituted by halogen atoms and lower alkyl groups. Examples of $R^a$, which is a constituent of formula (I-1), preferably include (1) linear or branched lower alkyl groups having about 1 to 5 carbon atoms such as methyl, ethyl, propyl, isopropyl, butyl, isobutyl, and pentyl and (2) linear or branched lower alkenyl groups having about 3 to 5 carbon atoms such as allyl, crotyl, methallyl, and 2-pentenyl.

Production of compounds (I-1)

1) Method of synthesis

The compounds of formula (I-1) can be produced by an objectively appropriate method. Specifically, they can be produced by, for example, a method represented by the following equation (A)-1:

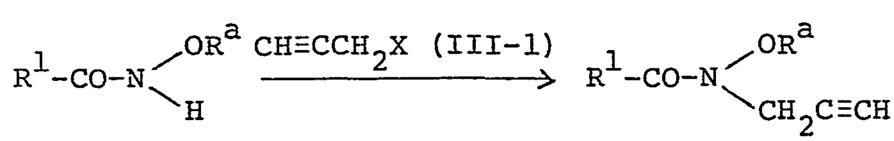


(II-1) (I-1)

[wherein $R^1$ and $R^a$ are the same as those defined in formula (I-1), and X stands for a halogen atom (particularly, chlorine, bromine or iodine)].

The reaction of the compounds of formula (II-1) with the compounds of formula (III-1) in accordance with

equation (A-1) can be conducted by allowing the compounds of formula (III-1) of at least equal mol per mol of the compounds of formula (II-1) to react therewith in the presence of a deacidification agent.

Examples of solvents which can be used include water, acetone, methanol, ethanol, and tetrahydrofuran. Preferable solvents are water, methanol, ethanol, and mixtures thereof.

Examples of deacidification agents are sodium alkoxides such as sodium methoxide and sodium ethoxide, alkali metal hydroxides such as sodium hydroxide and potassium hydroxide, and alkali metal carbonates such as sodium hydroxide and potassium carbonate. These can be used singly or as mixtures thereof. The amount of the deacidification agent used in mols is at least equal to that of the compound of formula (III-1).

The reaction is ordinarily conducted at a temperature of 0 - 100°C, preferably from room temperature to 50°C, with stirring for a period of from 1 hr to 3 days.

2) Purification method, starting materials, etc.

The compounds produced by the above mentioned method can be easily isolated for purification by an ordinary separation method such as solvent extraction, distillation, recrystallization, and silica gel column chromatography.

The compounds of formula (III-1) used as starting materials in the above mentioned equation (A) are well-known ones. Also, the compounds of formula (II-1) as

starting materials can be produced on the basis of, for example, the known process (Journal of Organic Chemistry, Vol. 25, p. 1734, 1960).

Compounds (I-2): Definition

The compound (I-2) of this invention are N-alkenyl-N-acylhydroxylamine derivatives represented by the following formula:

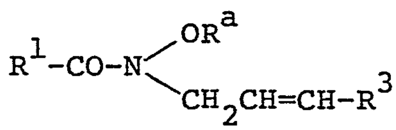

(I-2)

[wherein $R^1$, $R^a$ and $R^3$ are the same as those defined in the combination (2) of $R^o$, $R^a$, and $R^b$ in the definition of the compounds of the formula (A).]

Specific examples of $R^1$ and $R^a$ are the same as those enumerated for compounds (I-1). $R^3$ is hydrogen or a methyl group as set forth above.

Production of compounds (I-2)

1) Method of synthesis

The compounds of formula (I-2) can be produced by an objectively appropriate method. Specifically, they can be produced by, for example, a method represented by the following equation (A-2):

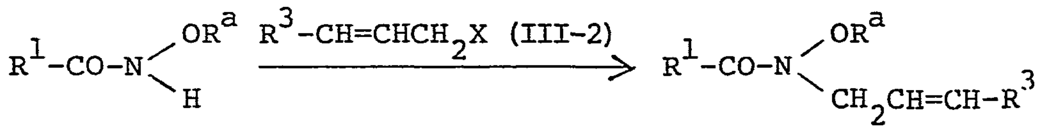


(II-2) (I-2)

[wherein $R^1$, $R^a$ and $R^3$ are the same as those defined

for formula (I-2), and X stands for a halogen atom (particularly chlorine, bromine, or iodine)].

The reaction of the compounds of formula (II-2) with the compounds of formula (III-2) in accordance with equation (A-2) can be conducted by allowing the compounds of formula (III-2) of a quantity in mols at least equal to the quantity of the compound of formula (II-2) to react therewith in the presence of a deacidification agent in solvents.

Examples of solvents, deacidification agent, reaction conditions, etc., are the same as those set forth with respect to the equation (A-1).

2) Purification, starting materials, etc.

Those set forth with respect to said equation (A-1) can be also applied here.

<u>Compounds (I-3): Definition</u>

Compounds (I-3) are N-alkenyl-N-acyl-O-alkenyl-hydroxylamine derivatives which are represented by the following formula, some of which are publicly known.

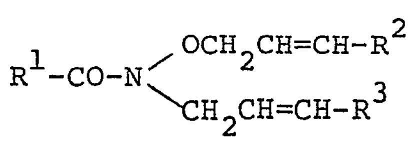

(I-3)

[wherein $R^1$ is the same as that set forth in the definition of the compounds of formula (A), and $R^2$ and $R^3$ are hydrogen and a methyl group respectively.]

<u>Production of Compounds (I-3)</u>

Some of the compounds of the formula (I-3) and the

production method thereof have been known from published literature [J. H. Cooley et al, Journal of Organic Chemistry, Vol. 25, p.1734 (1960)]. However, a number of compounds are novel and can be produced by an objectively appropriate method.

Specifically, they can be produced by, for example, a process indicated by the following equation (A-3):

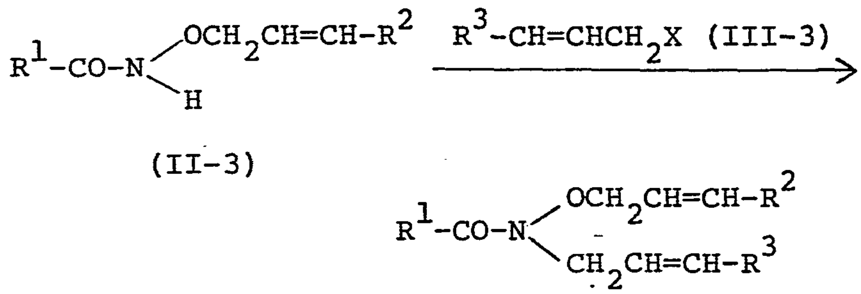


(II-3)

(I-3)

[wherein $R^1$, $R^2$ and $R^3$ are the same as those set forth with respect to formula (I-3), and X stands for a halogen atom (particularly chlorine, bromine, or iodine)].

The reaction of the compounds of the formula (II-3) with the compounds of the formula (III-3) in accordance with the equation (A-3) in the presence of a deacidification agent in solvents can be conducted by allowing the compounds of formula (III-3) in a quantity in mols at least equal to that of the compound of formula (II-3) to react therewith.

As examples of solvents, deacidification agents, reaction conditions, purification methods, etc., those set forth with respect to the equation (A-1) can be also applied here.

Compounds (I-4): Definition

The compounds (I-4) of this invention are hydroxylamine derivatives or salts thereof which are represented by the following formula:

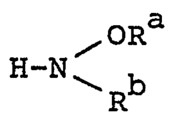

(I-4)

[wherein $R^a$ and $R^b$ are the same as those defined in the combination (4) of $R^o$, $R^a$ and $R^b$ in the definition of the compounds of the formula (A)].

Examples of $R^a$, which is a constituent in the formula (I-4), are preferably: (1) linear or branched lower alkyl groups having about 2 to 5 carbon atoms such as ethyl, n-propyl, n-butyl, isobutyl and n-pentyl; (2) linear or branched lower alkenyl groups having about 3 to 5 carbon atoms such as allyl, crotyl methallyl, and 2-pentenyl; and (3) linear or branched lower alkynyl having about 3 to 5 carbon atoms such as propargyl and 2-butynyl. Examples of $R^b$ which is a constituent in the formula (I-4) are preferably (1) linear or branched lower alkenyl groups having about 3 to 5 carbon atoms such as allyl, crotyl, methallyl and 2-pentenyl, and (2) linear or branched lower alkynyl groups having about 3 to 5 carbon atoms such as propargyl and 2-butynyl.

According to this invention, the salts of the compounds of formula (I-4) are also offered. Examples of the salts are those of inorganic acids such as hydrochloric

acid, hydrobromic acid, sulfuric acid, nitric acid, and phosphoric acid, and those of organic acids such as acetic acid, propionic acid, oxalic acid, citric acid, tartaric acid, lactic acid, and p-toluenesulfonic acid. Of these examples, pharmacologically allowable salts are particularly suitable.

Production of Compounds (I-4)

1) Method of synthesis

The compounds of formula (I-4) can be produced by an objectively appropriate method. Specifically, they can be produced by, for example, a method represented by the following equation (A-4):

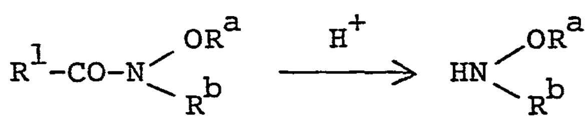


(II-4) (I-4)

[wherein $R^1$, $R^a$ and $R^b$ are the same as those defined hereinbefore.]

Hydrolyzing-reaction of the compounds of formula (II-4) in accordance with equation (A-4) can be conducted in the presence of acids in solvents.

Examples of solvents which may be used are water, acetone, methanol, ethanol, and tetrahydrofuran. Preferable solvents are water, methanol, ethanol, and mixtures thereof.

Examples of acids which may be used are inorganic acids such as hydrochloric acid, hydrobromic acid,

sulfuric acid, nitric acid, and phosphoric acid, and organic acids such as acetic acid, trifluoroacetic acid, and p-toluenesulfonic acid.

The reaction is ordinarily conducted at a temperature of 0 - 100°C, preferably from room temperature to 70°C, for a period of from 1 hr to one day with stirring.

2) Purification, starting materials, etc.

Those set forth with respect to the equation (A-1) can be also applied here.

The compounds of formula (II-2) which can be used as a starting material in the equation (A-1) can be produced by the method for producing the compounds (I-1) and by the method for producing the compounds (I-5) which will be described later or by processes on the basis of these methods.

<u>Compounds (I-5): Definition</u>

The compound (I-5) of this invention are N,O-dipropargyl-N-acylhydroxylamine derivatives which are represented by the following formula:

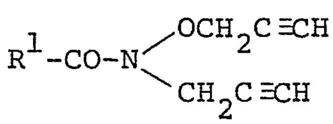

(I-5)

[wherein $R^1$ is the same as that defined in the combination (5) of $R^o$, $R^a$ and $R^b$ in the definition of the compounds of the formula (A).]

Specific examples of $R^1$ are the same as those set forth with respect to compounds (I-1).

Production of Compounds (I-5)

The compounds of formula (I-5) can be synthesized in accordance with an objectively appropriate method with respect to forming or converting of each bond.

Some preferable synthesizing methods are as follows.

1) Method of synthesis A-5

This method A-5 is conducted in accordance with the following equation (A-5).

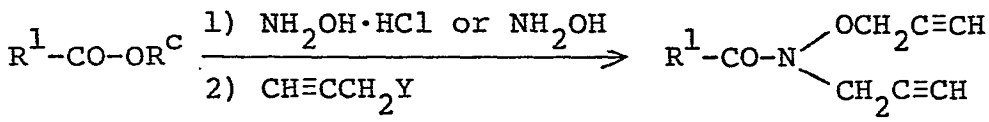


(II-5) (III-5) (I-5)

[wherein $R^1$ is the same as that set forth above; $R^c$ is a lower alkyl group (particularly, one having about 1 to 5 carbon atoms); and Y designates a halogen atom (particularly, chlorine, bromine, and iodine).]

Hydroxylamine source compounds, i.e., hydroxylamine free bases or salts in an amount preferably of 1.0 to 1.2 mol per mol of the compounds represented by formula (II-5) in equation (A-5) are caused to react with the compounds of formula (II-5), and then the compounds of formula (III-5) of at least 2 mol per mol of the compounds of formula (II-5) are caused to react therewith to produce the compounds of formula (I-5) of this invention.

It is desirable that the reaction represented by equation (A-5) be ordinarily carried out in solvents. Examples of solvents which can be used are such solvents

that can be used commonly in the reaction of the compounds of formula (II-5) with hydroxylamine source compounds and in the reaction of the compounds of formula (II-5) with compounds of formula (III-5) at the later stage. Specific examples are water, acetone, methanol, ethanol, and tetrahydrofuran. Particularly preferable solvents are alcohols such as methanol and ethanol, and mixtures thereof.

The net reaction at the first stage of equation (A-5) is the reaction of the compounds of formula (II-5) with hydroxylamines. Accordingly, the hydroxylamines can be in the form of salts with various kinds of acids as well as in the form of free bases. From this point of view, the compounds in both the forms are called hydroxylamines source compounds in this invention. Examples of acids which can form salts in this case are hydrochloric acid, phosphoric acid and other inorganic acids, and oxalic acid and other organic acids.

It is preferable to use alkalis in the condensation reaction of the compounds of formula (II-5) with hydroxylamines. In this case, the alkalis can be used not only for condensation reaction but also as a deacidification agent when a free base is prepared from hydroxylamine salts and also as a deacidification agent when the compounds of formula (III-5) at the later stage of the reaction are caused to react. Examples of the alkalis are alkali metal hydroxides such as sodium hydroxide and

potassium hydroxide, sodium alkoxides such as sodium methoxide and sodium ethoxide, alkali metal carbonates such as sodium carbonate and potassium carbonate.

In the case of the reaction of the compounds of formula (II-5) with hydroxylamine source compounds, sodium alkoxides or alkali metal hydroxides are ordinarily used with advantage. In the case of the reaction of the compounds of formula (II-5) with the compounds of formula (III-5) at a later stage, alkali metal hydroxides or alkali metal carbonates are ordinarily used with advantage. The alkalis to be used are desirably used in an amount of at least 3 mols in total per mol of the compounds of formula (II-5) in the case where there are intended to be generated from hydroxylamine salts free-bases thereof to continuously cause successive reaction of the compounds of formula (II-5). In the case where previously prepared hydroxylamine free bases are caused to react with the compounds of formula (II-5), at least 2 mols of alkalis per mol of the compounds of formula (II-5) are desirably used. In the above reaction, the first stage reaction of the compounds of formula (II-5) with hydroxylamine source compounds is ordinarily conducted at a temperature of about 0 - 50°C, preferably at room temperature, for a period of about 1 - 24 hours with stirring. The later stage reaction of the compounds of formula (II-5) with the compounds of formula (III-5) is ordinarily conducted at a temperature of about 0 -

100°C, preferably of from room temperature to 50°C, for a period of about from 1 hr to 3 days with stirring.

2) Method of synthesis B-5

The compounds of this invention can also be produced by a method represented by equation (B-5).

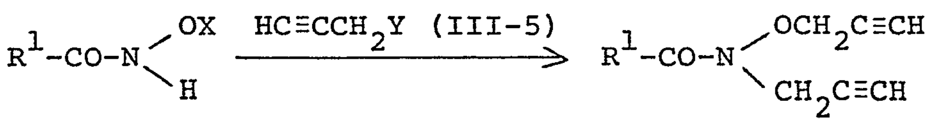


(IV-5) (I-5)

[wherein $R^1$ and Y are as set forth above, and X stands for a hydrogen atom or alkali metals.]

The reaction of the compounds of formula (IV-5) with the compounds (III-5) in accordance with equation (B-5) can be conducted by causing the compounds of formula (III-5) of at least 2 mols per mol of the compounds of formula (IV-5) to react therewith in the presence of a dioxidizer in a solvent.

Examples of solvents which can be used are water, acetone, methanol, ethanol, and tetrahydrofuran. Preferable solvents are water, methanol, ethanol, and mixtures thereof.

Examples of deacidification agents which can be used are sodium alkoxides, alkali metal hydroxides, and alkali metal carbonates. Particularly, alkali metal hydroxides such as sodium hydroxide and potassium hydroxide or alkali metal carbonates such as sodium carbonate, and potassium carbonate or mixtures of the hydroxides and carbonates

are preferably used.

The amount of the deacidification agent to be used is required to be at least 2 mols per mol of the compounds of formula (IV-5) in the case where the agent is used when X stands for a hydrogen atom in formula (IV-5). However, in the case where the deacidification agent is used when X stands for an alkali metal atom in formula (IV-5), the amount of the agent to be used is required to be at least one mol per mol of the compounds of formula (IV-5).

The reaction is ordinarily conducted at a temperature of about 0 - 100°C, preferably from room temperature to 50°C, for a period of from 1 hr to 3 days with stirring.

3) Method of synthesis C-5

Compounds (I-5) can be also produced by a method represented by equation (C-5).

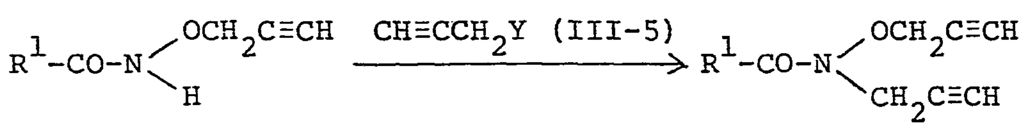


(V-5) (I-5)

[wherein $R^1$ and Y are as set forth above.]

The reaction of the compounds of formula (V-5) with the comounds of formula (III-5) in accordance with equation (C-5) can be conducted by allowing the compounds of formula (III-5) in a quantity of at least one mol per mol of the compounds of formula (V-5) to react therewith

in the presence of a deacidification agent in solvents.

Examples of solvents which can be used are water, acetone, methanol, ethanol, and tetrahydrofuran. Preferable solvents are water, methanol, ethanol and mixtures thereof.

Examples of deacidification agents which can be used are sodium alkoxides such as sodium methoxide and sodium ethoxide, alkali metal hydroxides such as sodium hydroxide and potassium hydroxide, and alkali metal carbonates such as sodium carbonate and potassium carbonate. These deacidification agents may be used alone or as mixtures thereof. The amount of a deacidification agent used is at least one mol per mol of the compounds of formula (V-5).

The reaction is ordinarily conducted at a temperature of 0 - 100°C, preferably from room temperature to 50°C, for a period of from 1 hr to 3 days with stirring.

4) Purification, starting material, etc.

Those set forth with respect to equation (A-1) can be also applied here.

The compounds of formula (II-5) and the compounds of formula (III-5) which can be used as starting materials are known compounds. The compounds of formula (IV-5) which can be used as starting materials in equation (B-5) are also known compounds. On the other hand, the compounds of formula (V-5) which can be used as starting materials in equation (C-5) can be produced by, for example, the

known method of Moore et al [Journal of Medical Chemistry, Vol. 12, p.45 (1969)].

Utilization of Compounds

1) Problems due to parasitosis

Parasitosis is prevalent in domestic animals such as pig, horse, cattle, sheep, goat, dog, cat, etc. and poultry such as chicken and is economically a serious problem.

As endoparasites of animals, various parasites have been known, examples of which are Taeniidae Cestoda in dog, cat, goat, horse and chicken; Ascaris in horse, pig, dog, cat and chicken; Oxyuris equi; Rhabditoidea in horse, pig, sheep and goat; Trichuris in pig, sheep and dog; Strongylus in horse; Oesophagostomum in cattle, pig, sheep and goat; Ancylostoma in cattle, sheep and dog; stomach worm in cattle, horse, sheep and goat; Ciliophora and cecum worm in chicken, turkey and duck; Filaria in cattle, horse, sheep, goat and dog; Fasciola hepatica parasitic in liver of cattle, horse, sheep, goat, pig, dog and cat; and Tramatode such as Echinostoma parasitic in digestive organ system. Because of infections caused by these parasites, the infected animals suffer from anemia, dystrophy, infirmity, reduction in body weight, and damage in stomach or colon tube walls and other tissues or organs. Thus, such infection is one of the causes of lowering of feed efficiency and lowering of productivity, thereby giving rise to a great economical loss.

2) Anthelmintic of the present invention

The compounds of the formula (A') have very high anthelmintic activities against a wide scope of various parasites as enumerated above.

So far as safety is concerned, all of the compounds of the formula (A') have very low toxicity. That is, no abnormality is observed when these compounds are administered in a dose of, for example, 700 mg to 1,100 mg/Kg for mouse, 700 to 1,000 mg/Kg for rat and 1,500 mg/Kg for chicken. Thus, these compounds are highly safe. (These values differ somewhat depending on the kind of the compound employed.)

Examples of the animals for which the compounds represented by the formula (A') are practically applicable are domestic animals such as pig, sheep, horse, cattle, goat, dog and cat and poultry such as chicken, turkey and duck. Furthermore, these compounds can be administered at any stage of growth of such animals, that is, when they are young, during growth, or after they are fully grown.

The dosage of the compounds of the formula (A') to be administered depends on the compound actually employed and the body weight of the animal to which the compound is administered, but, in general, a dose of over 0.1 mg/Kg per day, preferably between 1 mg/Kg and 100 mg/Kg, is administered in order to achieve effective results.

In the administration of the compounds of the formula

(I), they can be administered directly without addition of other components, or alternatively they can be administered as mixtures with physiologically nontoxic solid carriers or liquid carriers. Further, it is also possible to administer the compounds of the formula (I) by adding them directly into the feed or drinking water for the animals, or by adding the above mixtures with suitable carriers directly to the animals or into their feed or drinking water. The solid carriers employed in this case are, for example, orally digestable vessels such as gelatin capsules, or crude wheat powder, corn starch, defatted rice bran, calcium carbonate, calcium phosphate, talc, kaolin, white earth, lactose, sucrose, gelatin, stearic acid, agar, pectin and analogues thereof and also may be any of various excipients conventionally used in pharmaceuticals. As liquid vehicles, for example, nontoxic liquids such as water, isotonic sodium chloride solution, paraffins (e.g., petroleum distillates), vegetable oils (e.g., peanut oil, soybean oil or sesame oil), alcohols, (e.g., ethyl alcohol or glycerol), and glycols (e.g., propylene glycol or polyethylene alcohol) can be employed. Other auxiliary agents or additives such as emulsifiers, dispersants, suspending aids, humectants, etc. can also be used, depending on the necessity.

In practicing destruction of parasites by adding a compound of the formula (A'), it is compounded in a feed

(or drinking water) in a proportion of more than 1 ppm preferably 10 ppm to 500 ppm, before being administered to an animal. On the other hand, when the compound of (I) is to be administered without addition to a feed (or drinking water), it is possible to use various forms of preparations.

In the case of administration by the use of solid carriers, it is possible to administer the compound in a form such as a tablet, capsule, pellet, giant pill, or powder. Alternatively, it can be administered by using a liquid carrier in the form of a soft gelatin capsule or a suspension.

Further, when the compound of the formula (A') is dissolved or dispersed in a liquid carrier, it can be administered parenterally to an animal by injecting subcutaneously, intramuscularly, intravenously or intraperitoneally. In the case of parenteral administration, the compound of the present invention can be administered according to an aqueous recipe by the use of a vegetable oil such as peanut oil or soybean oil, or alternatively an aqueous parenteral recipe by the use of a watersoluble vehicle such as glycerol or polyethylene glycol can also be used. These recipes generally contain the compound of the formula (A') in an amount of 0.1 to 30% by weight.

Experimental Examples

The present invention is further described in detail by way of the following experimental examples. It is to be understood that the invention is not restricted to these examples, which are presented as illustrative only.

1) Syntheses A

Synthesis Example A1

A mixture of 1.3 g of N-acetyl-O-methylhydroxylamine, 0.82g of potassium hydroxide, 5 ml of methanol containing 10% water and 1.73 g of propargyl bromide was stirred for two days at room temperature. The solvent was removed therefrom under reduced pressure. Water was added thereto. The resulting mixture was subjected to extraction with ethyl acetate, dried with magnesium sulfate, concentrated and then purified by silica gel-column chromatography to produce 0.6 g of N-propargyl-N-acetyl-O-methylhydroxylamine (Compound No.A1) as a pale yellow oily substance.

Synthesis Example A2

A mixture of 3.9 g of N-acetyl-O-ethylhydroxylamine, 2.12 g of potassium hydroxide, 14 ml of methanol containing 10% water and 4.52 g of propargyl bromide was subjected to the same procedure as in Synthesis Example A1 to produce 2.4 g of N-propargyl-N-acetyl-O-ethylhydroxylamine (Compound A2) as a pale yellow oily substance.

Synthesis Example A3

A mixture of 30.8 g of N-acetyl-O-allylhydroxylamine, 15 g of potassium hydroxide, 160 ml of methanol containing 10% water and 32.5 g of propargyl bromide was subjected to the same procedure as in Synthesis Example A1 except that purification was carried out by distillation under reduced pressure to produce 22.3 g of N-propargyl-N-acetyl-O-allylhydroxylamine (Compound A3); boiling point 74 - 76°C/2 mmHg.

Synthesis Example A4

A mixture of 3.87 g of N-acetyl-O-crotylhydroxylamine, 1.2 g of sodium hydroxide, 15 ml of methanol containing 10% water and 3.64 g of propargyl bromide was subjected to the same procedure as in Synthesis Example A1 to prepare 2.62 g of N-propargyl-N-acetyl-O-crotylhydroxylamine (Compound No.A4).

Synthesis Example A5

A mixture of 3.87 g of N-propionyl-O-allylhydroxylamine, 1.77 g of potassium hydroxide, 15 ml of methanol containing 10% water and 2.28 g of propargyl chloride was subjected to the same procedure as in Synthesis Example A1 to prepare 2.27 g of N-propargyl-N-propionyl-O-allylhydroxylamine (Compound No.A5).

Synthesis Example A6

A mixture of 4.29 g of N-butylyl-O-allylhydroxylamine, 1.77 g of potassium hydroxide, 15 ml of ethanol containing 10% water and 3.64 g of propargyl bromide was subjected

to the same procedure as in Synthesis Example A1 to produce 4.37 g of N-propargyl-N-butylyl-O-allylhydroxylamine (Compound No.A6).

Synthesis Example A7

A mixture of 4.0 g of N-valeroyl-O-allylhydroxylamine, 1.43 g of potassium hydroxide, 10 ml of methanol containing 10% water and 3.1 g of propargyl bromide was subjected to the same procedure as in Synthesis Example A1 to produce 4.54 g of N-propargyl-N-valeroyl-O-allylhydroxylamine (Compound No.A7).

Synthesis Example A8

A mixture of 3.37 g of N-oleoyl-O-allylhydroxylamine, 0.53 g of sodium methoxide, 10 ml of methanol and 1.31 g of propargyl bromide was subjected to the same procedure as in Synthesis Example A1 to prepare 2.94 g of N-propargyl-N-oleoyl-O-allylhydroxylamine (Compound No.A8).

Synthesis Example A9

A mixture of 3.39 g of N-stearoyl-O-allylhydroxylamine, 0.53 g sodium methoxide, 10 ml of methanoltetrahydrofuran (1:1) mixture and 1.31 g of propargyl bromide was subjected to the same procedure as in Synthesis Example A1 to prepare 3.05 g of N-propargyl-N-stearoyl-N-allylhydroxylamine (Compound No.A9).

Synthesis Example A10

A mixture of 48.3 g of N-benzoyl-O-butylhydroxylamine, 14 g of potassium hydroxide, 260 ml of methanol containing 20% water and 29.8 g of propargyl bromide was subjected to

the same procedure as in Synthesis Example A1 except that purification was carried out by distillation under reduced pressure to produce 42.3 g of N-propargyl-N-benzoyl-O-butylhydroxylamine (Compound A10) of a boiling point of 110 - 111°C (0.2 mmHg).

Synthesis Example A11

A mixture of 17.7 g of N-benzoyl-O-allylhydroxylamine, 5.9 g of potassium hydroxide, 100 ml of methanol containing 10% water and 12 g of propargyl bromide was subjected to the same procedure as in Synthesis Example A1 except that purification was carried out by distillation under reduced pressure to produce 16.5 g of N-propargyl-N-benzoyl-O-allylhydroxylamine (Compound A11) of a boiling point of 128 - 130°C (0.5 mmHg).

Synthesis Example A12

A mixture of 3.9 g of N-(p-fluorobenzoyl)-O-allylhydroxylamine, 1.06 g of potassium hydroxide, 20 ml of methanol containing 10% water and 1.2 g of propargyl bromide was subjected to the same procedure as in Synthesis Example A1 to prepare 3.5 g of N-propargyl-N-(p-fluorobenzoyl)-O-allylhydroxylamine (Compound A12).

Synthesis Example A13

A mixture of 3.03 g of N-formyl-O-allylhydroxylamine, 1.77 g of potassium hydroxide, 15 ml of methanol containing 10% water and 3.64 g of propargyl bromide was subjected to the same procedure as in Synthesis Example A1 to prepare 0.9 g of N-propargyl-N-formyl-O-allylhydroxyl-

amine (Compound A13).

The spectral values of representative compounds of the formula (1) obtained in Synthesis Examples A1 through A13 are shown in Table A1.

Table A1

| Compound Nos. | 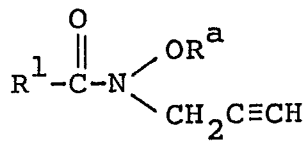 | | NMR (δ, ppm) | |
|---|---|---|---|---|
| | $R^1$ | $R^2$ | ≡CH | $-N-CH_2-$ |
| A1 | $CH_3-$ | $-CH_3$ | 2.30 | 4.40 |
| A2 | $CH_3-$ | $-C_2H_5$ | 2.26 | 4.40 |
| $A_3$ | $CH_3-$ | $-CH_2CH=CH_2$ | 2.33 | 4.40 |
| $A_4$ | $CH_3-$ | $-CH_2CH=CHCH_3$ | 2.32 | 4.40 |
| A5 | $C_2H_5-$ | $-CH_2CH=CH_2$ | 2.27 | 4.40 |
| A6 | $^nC_3H_7-$ | $-CH_2CH=CH_2$ | 2.27 | 4.40 |
| A7 | $^nC_4H_9-$ | $-CH_2CH=CH_2$ | 2.25 | 4.40 |
| A8 | $C_{17}H_{33}-$ | $-CH_2CH=CH_2$ | 2.18 | 4.41 |
| A9 | $C_{17}H_{35}-$ | $-CH_2CH=CH_2$ | 2.18 | 4.41 |
| A10 | 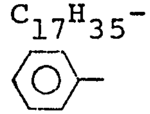 | $-nC_4H_9$ | 2.36 | 4.53 |
| A11 | 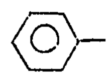 | $-CH_2CH=CH_2$ | 2.37 | 4.52 |
| A12 | F- 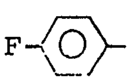 | $-CH_2CH=CH_2$ | 2.33 | 4.53 |
| A13 | H — | $-CH_2CH=CH_2$ | 2.50 | 4.60 |

These Compound numbers will be referred to in the description below.

1) Syntheses B

Synthesis Example B1

A mixture of 17.8 g of N-acetyl-O-methylhydroxylamine, 11.2 g of potassium hydroxide, 100 ml of methanol containing 10% water and 25.4 g of allyl bromide was stirred for two days at room temperature. The solvent was removed therefrom under reduced pressure. Water was added thereto. The resulting mixture was subjected to extraction with ethyl acetate, dried with magnesium sulfate, concentrated and then purified by distillation under reduced pressure to produce 7.2 g of N-allyl-N-acetyl-O-methylhydroxylamine (Compound No.B1) as an oily substance of a boiling point of 63 - 65°C (20 mmHg).

Synthesis Example B2

A mixture of 3.09 g of N-propionyl-O-methylhydroxylamine, 1.74 g of potassium hydroxide, 10 ml of methanol containing 10% water and 3.87 g of allyl bromide was subjected to the same procedure as in Synthesis Example B1 except that purification was carried out through silica gel-column chromatography to prepare 2.04 g of N-allyl-N-propionyl-O-methylhydroxylamine (Compound B2) as an oily substance.

Synthesis Example B3

A mixture of 3.93 g of N-butylyl-O-methylhydroxylamine, 1.2 g of potassium hydroxide, 10 ml of methanol containing 10% water and 2.53 g of allyl chloride was subjected to the same procedure as in Synthesis Example B1

except that purification was carried out through silica gel-column chromatography to prepare 2.18 g of N-allyl-N-butylyl-O-methylhydroxylamine (Compound B3).

Synthesis Example B4

A mixture of 6.22 g of N-oleoyl-O-methylhydroxylamine, 1.08 g of sodium methoxide, 20 ml of a methanol/tetrahydrofuran (1:1) mixture, and 2.66 g of allyl bromide was subjected to the same procedure as in Synthesis Example B1 except that purification was carried out through silica gel-column chromatography to produce 5.97 g of N-allyl-N-oleoyl-O-methylhydroxylamine (Compound B4).

Synthesis Example B5

A mixture of 4.53 g of N-benzoyl-O-methylhydroxylamine, 1.74 g of potassium hydroxide, 10 ml of methanol containing 20% water and 3.88 g of allyl bromide was subjected to the same procedure as in Synthesis Example B1 except that purification was carried out through silica gel-column chromatography to prepare 4.36 g of N-allyl-N-benzoyl-O-methylhydroxylamine (Compound B5).

Synthesis Example B6

A mixture of 7.5 g of N-formyl-O-methylhydroxylamine, 5.6 g of potassium hydroxide, 40 ml of methanol containing 10% water and 13.3 g of allyl bromide was subjected to the same procedure as in Synthesis Example B1 except that purification was carried out through silica gel-column chromatography to produce 0.9 g of N-allyl-N-formyl-O-methylhydroxylamine (Compound B6).

0103895

Synthesis Example B7

A mixture of 2.67 g of N-acetyl-O-methylhydroxylamine, 1.74 g of potassium hydroxide, 10 ml of methanol containing 10% water and 4.46 g of crotyl bromide was subjected to the same procedure as in Synthesis Example B1 except that purification was carried out through silica gel-column chromatography to produce 2.20 g of N-crotyl-N-acetyl-O-methylhydroxylamine (Compound B7).

Synthesis Example B8

A mixture of 10.3 g of N-acetyl-O-ethylhydroxylamine, 5.6 g of potassium hydroxide, 50 ml of methanol containing 10 % water and 13.3 g of allyl bromide was subjected to the same procedure as in Synthesis Example B1 and purified by distillation under reduced pressure to produce 7.7 g of N-allyl-N-acetyl-O-ethylhydroxylamine (Compound B8) of a boiling point of 73 - 74°C (18 mmHg).

Synthesis Example B9

A mixture of 3.51 g of N-propionyl-O-ethylhydroxylamine, 1.2 g of sodium hydroxide, 10 ml of methanol and 3.87 g of allyl bromide was subjected to the same procedure as in Synthesis Example B1 except that purification was carried out through silica gel-column chromatography to produce 2.73 g of N-allyl-N-propionyl-O-ethylhydroxylamine (Compound B9).

Synthesis Example B10

A mixture of 2.62 g of N-butylyl-O-ethylhydroxylamine,

1.12 g of potassium hydroxide, 10 ml of methanol containing 10% water and 1.68 g of allyl chloride was subjected to the same procedure as in Synthesis Example B1 except that purification was carried out through silica gel-column chromatography to obtain 1.74 g of N-allyl-N-butylyl-O-ethylhydroxylamine (Compound B10).

Synthesis Example B11

A mixture of 2.90 g of N-isovaleroyl-O-ethylhydroxylamine, 1.12 g of potassium hydroxide, 10 ml of ethanol and 2.66 g of allyl bromide was subjected to the same procedure as in Synthesis Example B1 except that purification was carried out through silica gel-column chromatography, whereupon 2.44 g of N-allyl-N-isovaleroyl-O-ethylhydroxylamine (Compound B11) was obtained.

Synthesis Example B12

A mixture of 3.27 g of N-stearoyl-O-ethylhydroxylamine, 0.54 g of sodium methoxide, 10 ml of a methanol/tetrahydrofuran (1:1) mixture, and 1.33 g of allyl bromide was subjected to the same procedure as in Synthesis Example B1 except that purification was carried out through silica gel-column chromatography, whereupon 3.19 g of N-allyl-N-stearoyl-O-ethylhydroxylamine (Compound No.B12) was obtained.

Synthesis Example B13

A mixture of 3.30 g of N-benzoyl-O-ethylhydroxylamine, 1.12 g of potassium hydroxide, 15 ml of methanol containing

10% water and 2.66 g of allyl bromide was subjected to the same procedure as in Synthesis Example B1 except that purification was carried out through column chromatography to obtain 3.28 g of N-allyl-N-benzoyl-O-ethylhydroxylamine (Compound No.B13).

Synthesis Example B14

A mixture of 1.83 g of N-(p-fluorobenzoyl)-O-ethylhydroxylamine, 0.56 g of potassium hydroxide, 8 ml of methanol containing 10% water and 1.35 g of allyl bromide was subjected to the same procedure as in Synthesis Example B1 except that purification was carried out through column chromatography to obtain 1.83 g of N-allyl-N-(p-fluorobenzoyl)-O-ethylhydroxylamine (Compound No.B14).

Synthesis Example B15

A mixture of 8.9 g of n-formyl-O-ethylhydroxylamine, 5.6 g of potassium hydroxide, 40 ml of methanol containing 10% water and 13.3 g of allyl bromide was subjected to the same procedure as in Synthesis Example B1 except that purification was carried out through column chromatography to produce 1.55 g of N-allyl-N-formyl-O-ethylhydroxylamine (Compound No.B15).

Synthesis Example B16

A mixture of 3.09 g of N-acetyl-O-ethylhydroxylamine, 1.62 g of sodium methoxide, 10 ml of methanol and 2.99 g of crotyl bromide was subjected to the same procedure as in Synthesis Example B1 except that purification was carried

through column chromatography to produce 2.17 g of N-crotyl-N-acetyl-O-ethylhydroxylamine(Compound No.B16).

Synthesis Example B17

A mixture of 2.34 g of N-acetyl-O-propylhydroxylamine, 1.12 g of potassium hydroxide, 10 ml of methanol containing 10% water and 2.66 g of allyl bromide was subjected to the same procedure as in Synthesis Example B1 except that purification was carried out through column chromatography to produce 1.57 g of N-allyl-N-acetyl-O-propylhydroxylamine (Compound No.B17).

Synthesis Example B18

A mixture of 2.62 g of N-acetyl-O-butylhydroxylamine, 1.36 g of sodium ethoxide, 10 ml of ethanol and 2.66 g of allyl bromide was subjected to the same procedure as in Synthesis Example B1 except that purification was carried out through column chromatography to produce 1.98 g of N-allyl-N-acetyl-O-butylhydroxylamine (Compound No.B18).

Synthesis Example B19

A mixture of 9.7 g of N-benzoyl-O-butylhydroxylamine, 2.6 g of potassium hydroxide, 30 ml of methanol containing 10% water and 6.7 g of allyl bromide was subjected to the same procedure as in Synthesis Example B1 and purified by distillation under reduced pressure to produce 8.8 g of N-allyl-N-benzoyl-O-butylhydroxylamine (Compound B19) of a boiling point of 99 - 100°C (0.1 mmHg).

Synthesis Example B20

A mixture of 2.9 g of N-acetyl-O-valerylhydroxylamine, 1.12 g of potassium hydroxide, 10 ml of methanol and 2.66 g of allyl bromide was subjected to the same procedure as in Synthesis Example B1 except that purification was carried out through silica gel-column chromatography to produce 2.41 g of N-allyl-N-acetyl-O-valerylhydroxylamine (Compound No.B20).

The spectral values of representative compounds of the formula (I-2) obtained in Synthesis Examples B1 through B20 are shown in Table B1.

Table B1

| Compound Nos. | 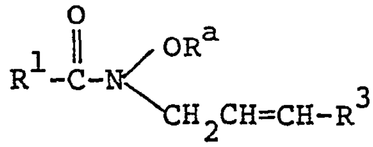 | | | N M R δ, (J, Hz) $-N-CH_2-$ |
|---|---|---|---|---|
| | $R^1$ | $R^2$ | $R^3$ | |
| B1 | $CH_3-$ | $-CH_3$ | -H | 4.25(d,6) |
| B2 | $C_2H_5-$ | $-CH_3$ | -H | 4.25(d,6) |
| B3 | $nC_4H_9-$ | $-CH_3$ | -H | 4.25(d,6) |
| B4 | $C_{17}H_{33}-$ | $-CH_3$ | -H | 4.23(d,6) |
| B5 | 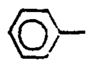 | $-CH_3$ | -H | 4.36(d,7) |
| B6 | H— | $-CH_3$ | -H | 4.25(d,6) |
| B7 | $CH_3-$ | $-CH_3$ | $-CH_3$ | 4.18(d,6) |
| B8 | $CH_3-$ | $-C_2H_5$ | -H | 4.25(d,6) |
| B9 | $C_2H_5-$ | $-C_2H_5$ | -H | 4.25(d,6) |
| B10 | $nC_3H_7-$ | $-C_2H_5$ | -H | 4.25(d,6) |
| B11 | $iC_4H_9-$ | $-C_2H_5$ | -H | 4.24(d,6) |
| B12 | $C_{17}H_{35}-$ | $-C_2H_5$ | -H | 4.23(d,6) |
| B13 | 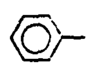 | $-C_2H_5$ | -H | 4.35(d,7) |
| B14 | 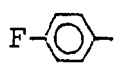 | $-C_2H_5$ | -H | 4.37(d,7) |
| B15 | H— | $-C_2H_5$ | -H | 4.25(d,6) |
| B16 | $CH_3-$ | $-C_2H_5$ | $-CH_3$ | 4.18(d,6) |
| B17 | $CH_3-$ | $-nC_3H_7$ | -H | 4.25(d,6) |
| B18 | $CH_3-$ | $-nC_4H_9$ | -H | 4.24(d,6) |
| B19 | 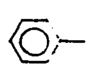 | $-nC_4H_9$ | -H | 4.35(d,7) |
| B20 | $CH_3-$ | $-nC_5H_{11}$ | -H | 4.24(d,6) |

These compound numbers will be referred to in the description below.

1) Syntheses C

Synthesis Example C1

A mixture of 23 g of N-acetyl-O-allylhydroxylamine, 11.2 g of potassium hydroxide, 100 ml of methanol containing 10% water and 25.4 g of allyl bromide was stirred for 2 days at room temperature. The solvent was removed under reduced pressure, and water was added thereto. The resulting mixture was subjected to extraction with ethyl acetate, dried with magnesium sulfate, concentrated and purified by distillation under reduced pressure to produce 17.4 g of N,O-diallyl-N-acetylhydroxylamine (Compound No.C1) of a boiling point of 47 - 48°C (1 mmHg);NMR, δ, 4.25 (d, J = 6 Hz, N-$CH_2$-).

Synthesis Examples C2 through C10

The compounds represented by the formula (I-3) given above and listed in the table C1 below can be produced according to the same procedure as in Synthesis Example C1.

Table C1

| Synthesis Example Nos. | Compound Nos. | $R^1$ | $R^a$ | $R^3$ | N M R δ, (J) $-N-CH_2-$ |
|---|---|---|---|---|---|
| C2 | C2 | $C_2H_5-$ | H- | H- | 4.25(d, 6) |
| C3 | C3 | $nC_3H_7-$ | H- | H- | 4.24(d, 6) |
| C4 | C4 | $C_{17}H_{33}-$ | H- | H- | 4.23(d, 6) |
| C5 | C5 | $C_{17}H_{35}-$ | H- | H- | 4.23(d, 6) |
| C6 | C6 | 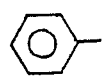 | H- | H- | 4.39(d, 7) |
| C7 | C7 | 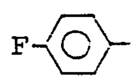 | H- | H- | 4.42(d, 7) |
| C8 | C8 | H- | H- | H- | 4.25(d, 6) |
| C9 | C9 | $CH_3-$ | H- | $CH_3-$ | 4.18(d, 6) |
| C10 | C10 | $CH_3-$ | $CH_3-$ | $CH_3-$ | 4.18(d, 6) |

These compound numbers will be referred to in the description below.

1) Syntheses D

Synthesis Example D1

A mixture of 3.62 g of N-allyl-N-acetyl-O-ethylhydroxylamine, 10 ml of ethanol and 15 ml of 6N hydrochloric acid was stirred for one hour at 70°C.

After the ethanol used as a solvent and water were removed from the mixture under reduced pressure, the product was dried to produce 3.40 g of white crystalline N-allyl-O-ethylhydroxylamine hydrochloride (Compound No.D1) of a melting point of 37°C.

Synthesis Example D2

After a mixture of 21.9 g of N-allyl-N-benzoyl-O-propylhydroxylamine, 50 ml of ethanol and 50 ml of 6N hydrochloric acid was stirred for 8 hours at 70°C, ethanol was removed therefrom under reduced pressure. The resulting concentrated aqueous solution was washed twice with ethyl acetate. Subsequently, the aqueous layer was made alkaline with sodium hydroxide, which step was followed by extraction with methylene chloride. The resulting organic layer was dried with sodium sulfate, and the solvent was removed therefrom under reduced pressure to give an oily substance, which was then purified by distillation under reduced pressure to yield 4.62g of N-allyl-O-propylhydroxylamine (Compound No. D2) of a boiling point of 37 - 38°C (10 mmHg).

Synthesis Example D3

A mixture of 3.92 g of N-propargyl-N-acetyl-O-ethylhydroxylamine, 10 ml of ethanol and 15 ml of 6N hydrochloric acid is subjected to the same procedure as in Synthesis Example D1 to obtain 3.70 g of N-propargyl-O-ethylhydroxylamine hydrochloride (Compound No.D10) as pale yellow crystals of a melting point of 85 - 86°C.

Synthesis Example D4

After a mixture of 15.3 g of N-propargyl-N-acyl-O-allylhydroxylamine, 50 ml of ethanol and 50 ml of 6N hydrochloric acid was stirred for one hour at 70°C, ethanol was removed therefrom under reduced pressure. The resulting concentrated aqueous solution was made alkaline with sodium hydroxide, which step was followed by extraction with methylene chloride. The resulting organic layer was dried with sodium sulfate, and the solvent was removed therefrom under reduced pressure to produce an oily substance, which was then purified by distillation under reduced pressure to yield 4.66 g of N-propargyl-O-allylhydroxylamine (Compound No.D13) of a boiling point of 49 - 50°C (13 mmHg).

Synthesis Example D5

From the concentrated aqueous solution obtained according to Synthesis Example D4, water is completely removed by additional concentration under reduced pressure and an azeotropic removal method by using benzene and ethanol to produce 14.5 g of N-propargyl-O-allyl-hydroxylamine hydrochloride (Compound No.D14) as an oily substance.

Synthesis Example D6

After a mixture of 4.40 g of N-propargyl-N-acetyl-O-hydroxylamine, 10 ml of ethanol and 15 ml of 6N hydrochloric acid is stirred for 45 minutes at 60°C, it is subjected to the same procedure as in Synthesis Example D1 to produce 4.06 g of white crystalline N-propargyl-O-methylhydroxylamine hydrochloride (Compound No.D16) of a melting point of 104 - 105°C.

The reaction procedure as in Synthesis Examples D1 through D6 is repeated to produce the compounds (Compound Nos. D3, D4, D5, D6, D7, D8, D9, D11, D12 and D15) listed in Table D1.

Table D1

| Compound Nos. | Structural formula | NMR, N-$CH_2$- δ, (J) | Solvent |
|---|---|---|---|
| D1 | $C_2H_5$-ONH-$CH_2$CH=$CH_2$·HCl | 3.98(d,6) | $D_2O$ |
| D2 | $^nC_3H_5$-ONH-$CH_2$CH=$CH_2$ | 3.51(d,6) | $CDCl_3$ |
| D3 | i-$C_4H_9$-ONH-$CH_2$CH=$CH_2$ | 3.50(d,6) | $CDCl_3$ |
| D4 | $CH_2$=CH$CH_2$-ONH-$CH_2$CH=$CH_2$ | 3.51(d,6) | $CDCl_3$ |
| D5 | HC≡C $CH_2$-ONH-$CH_2$CH=$CH_2$ | 3.60(d,6) | $CDCl_3$ |
| D6 | HC≡C $CH_2$-ONH-$CH_2$CH=$CH_2$·HCl | 3.85(dd,2,6) | $D_2O$ |
| D7 | $C_2H_5$-ONH-$CH_2$CH=CH-$CH_3$ | 3.44(d,6) | $CDCl_3$ |
| D8 | $CH_2$=CH$CH_2$-ONH-$CH_2$CH=CH-$CH_3$ | 3.53(d,6) | $CDCl_3$ |
| D9 | $CH_3$-ONH-$CH_2$CH=$CH_2$·HCl | 3.77(d,6) | $D_2O$ |
| D10 | $C_2H_5$-ONH-$CH_2$C≡CH·HCl | 4.12(d,2) | $D_2O$ |
| D11 | n-$C_3H_7$-ONH-$CH_2$C≡CH | 3.68(d,2) | $CDCl_3$ |
| D12 | n-$C_4H_9$-ONH-$CH_2$C≡CH | 3.67(d,2) | $CDCl_3$ |
| D13 | $CH_2$=CH$CH_2$-ONH-$CH_2$C≡CH | 3.68(d,2) | $CDCl_3$ |
| D14 | $CH_2$=CH$CH_2$-ONH-$CH_2$C≡CH·HCl | 4.10(d,2) | $D_2O$ |
| D15 | HC≡C $CH_2$-ONH-$CH_2$C≡CH·HCl | 4.07(d,2) | $D_2O$ |
| D16 | $CH_3$-ONH-$CH_2$C≡CH·HCl | 4.07(d,2) | $D_2O$ |

These compound numbers will be referred to in the description below.

1) Syntheses E

Synthesis Example E1

A solution of 4.18 of hydroxylamine hydrochloride in 50 ml of absolute methanol was added to 4.14 g of metallic sodium dissolved in 90 ml of absolute methanol, and then 5.28 g of ethyl acetate was added thereto. After stirring the resulting mixture for one day at room temperature, sodium chloride precipitate was filtered off, and the filtrate was concentrated under reduced pressure to about 70 ml. To the resulting solution were added 4.2 g of sodium carbonate and 20 ml of water, and then 15 g of propargyl bromide was added, and the resulting mixture was stirred for 3 days at room temperature. The reaction liquid was concentrated under reduced pressure and, after addition of water, was made acidic with concentrated hydrochloric acid to carry out extraction with chloroform. After the resulting chloroform layer was washed with a 10% aqueous sodium hydroxide solution, it was washed with water, dried with magnesium sulfate, concentrated and then distilled under reduced pressure to produce 5.5 g N,O-dipropargyl-N-acetylhydroxylamine (Compound No.E1) as a pale yellow oily substance.

Synthesis Example E2

A solution of 4.18 g of hydroxylamine hydrochloride in 50 ml of absolute methanol was added to 4.14 g of metallic sodium dissolved in 90 ml of absolute methanol, and then 5.28 g of ethyl acetate was added thereto.

After stirring the resulting mixture for one day at room temperature, sodium chloride precipitate was filtered off, and the filtrate was concentrated under reduced pressure to about 70 ml. To the resulting solution were added 4.2 g of sodium carbonate and 20 ml of water, and then 9.4 g of propargyl chloride was added. The resulting mixture was then subjected to the same procedure as in Synthesis Example E1 to produce 4.8 g of N,O-dipropargyl-N-acetylhydroxylamine (Compound No. E1).

Synthesis Example E3

A solution of 4.18 g of hydroxylamine hydrochloride in 50 ml of absolute methanol was added to a solution of 4.14 g of metallic sodium dissolved in 90 ml of absolute methanol, and then 5.28 g of methyl propionate was added thereto. Then the same procedure as in Synthesis Example E1 was repeated to produce 4.7g of N,O-dipropargyl-N-propionylhydroxylamine (Compound No.E2) as a pale yellow oily substance.

Synthesis Example E4

A solution of 4.18 g of hydroxylamine hydrochloride in 50 ml of absolute methanol was added to a solution of 4.14 g of metallic sodium dissolved in 90 ml of absolute methanol, and then 6.12 of methyl n-butyrate was added thereto. Then the same procedure as in Synthesis Example E1 was repeated to produce 5.7 g of N,O-dipropargyl-N-butylylhydroxylamine (Compound No.E3) as a pale yellow oily substance.

Synthesis Example E5

A solution of 4.18 g of hydroxylamine hydrochloride in 50 ml of absolute methanol was added to a solution of 4.14 g of metallic sodium dissolved in 90 ml of absolute methanol, and then 6.96 g of methyl valerate was added thereto. Then the same procedure as in Synthesis Example E1 was repeated to produce 6.5 g of N,O-dipropargyl-N-valerylhydroxylamine (Compound No.E4) as a pale yellow oil substance.

Synthesis Example E6

A solution of 4.18 g of hydroxylamine hydrochloride in 50 ml of absolute methanol was added to a solution of 4.14 g of metallic sodium dissolved in 90 ml of absolute methanol, and then 7.80 g of methyl n-caproate was added thereto. Then the same procedure as in Synthesis Example E1 was repeated to produce 7.0 g of N,O-dipropargyl-N-caproylhydroxylamine (Compound No.E5).

Synthesis Example E7

A solution of 4.18 g of hydroxylamine hydrochloride in 50 ml of absolute methanol was added to 4.14 g of metallic sodium dissolved in 90 ml of absolute methanol and then 16.9 g of methyl oleate was added thereto. The resulting mixture was stirred for one day at room temperature. Subsequently, to the mixture were added 4.2 g of sodium carbonate, 70 ml of tetrahydrofuran, 20 ml of water and 15 g of propargyl bromide, and the whole mixture was stirred for 4 days at room temperature. The mixture

was subjected to the same procedure as in Synthesis Example E1 and to silica gel-column chromatography for isolation and purification to produce 13.5 g of N,O-dipropargyl-N-oleoylhydroxylamine (Compound No.E6).

Synthesis Example E8

To a mixture of 15.2 g of sodium benzohydroxamate, 5.3 g of potassium hydroxide, 6.0 g of sodium carbonate, 95 ml of methanol and 60 ml of water was added 25 g of propargyl bromide, and the resulting mixture was stirred for two days at room temperature. After concentration, the reaction liquid was diluted with water and made acidic with conc. hydrochloric acid, which step was followed by extraction with chloroform. The chloroform layer thus obtained was washed with 10% sodium hydroxide solution and with water, dried with magnesium sulfate, concentrated and distilled under reduced pressure to produce 15.6 g of N,O-dipropargyl-N-benzoylhydroxylamine (Compound No.E7) as a pale yellow oily substance.

Synthesis Example E9

To a mixture of 2.92 g of propargyl-O-chlorobenzo-hydroxamate, 0.84 g of potassium hydroxide, 1.0 g of sodium carbonate, 15 ml of methanol and 2 ml of water was added 1.97 g of propargyl bromide, and the resulting mixture was stirred for two days at room temperature. Then, the resulting mixture was subjected to the same procedure as in Synthesis Example E8, which was followed by isolation and purification by silica gel-column

chromatography to produce 1.98 g of N,O-dipropargyl-N-O-chlorobenzoylhydroxylamine (Compound No.E8).

Synthesis Example E10

A solution of 4.18 g of hydroxylamine hydrochloride in 50 ml of absolute methanol is added to a solution of 4.14 g of metallic sodium dissolved in 90 ml of absolute methanol, and then 4.56 g of methyl formate is added thereto. Then the same procedure as in Synthesis Example E1 is repeated to produce 1.3 g of N,O-dipropargyl-N-formylhydroxylamine (Compound No.E9).

Synthesis Example E11

A solution of 4.18 g of hydroxylamine hydrochloride in 50 ml of absolute methanol is added to a solution of 4.14 g of metallic sodium dissolved in 90 ml of absolute methanol, and then 12.8 g of methyl laurate is added thereto. Then the same procedure as in Synthesis Example E7 is repeated to produce 8.4 g of N,O-dipropargyl-N-lauroylhydroxylamine (Compound No.E10) in the form of crystals.

Synthesis Example E12

A solution of 4.18 g of hydroxylamine hydrochloride in 50 ml of absolute methanol is added to a solution of 4.14 g of metallic sodium dissolved in 90 ml of absolute methanol, and 17.88 g of methyl stearate is added thereto. Then the same procedure as in Synthesis Example E7 is repeated to produce 7.8 g of N,O-dipropargyl-N-stearoylhydroxylamine (Compound No.E11) in the form of crystals.

The physical properties and spectral values of representative compounds of the present invention obtained in Synthesis Examples E1 through E12 are shown in Table E1.

Table E1

| Compound Nos. | 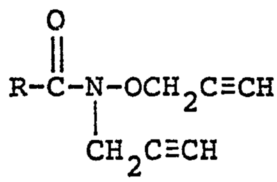<br>R- | bp, °C(mmHg)<br>mp, °C<br>$[n_D^{20}]$ | N M R<br>(δ, ppm)<br>≡CH |
|---|---|---|---|
| E1 | $CH_3$- | 72°(1) | 2.33 2.69 |
| E2 | $CH_3CH_2$- | 89-90°(1) | 2.28 2.63 |
| E3 | $CH_3(CH_2)_2$- | 73-75°(0.2) | 2.29 2.63 |
| E4 | $CH_3(CH_2)_3$- | 74-76°(0.2) | 2.28 2.63 |
| E5 | $CH_3(CH_2)_4$- | 80-83°(0.3) | 2.28 2.62 |
| E6 | $C_{17}H_{33}$- | [1.390] | 2.20 2.57 |
| E7 | 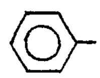 | 128-131°(1) | 2.37 2.52 |
| E8 | 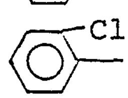 | [1.355] | 2.33 2.52 |
| E9 | H- | [1.380] | 2.30 2.63 |
| E10 | $C_{11}H_{23}$- | 38-39° | 2.20 2.56 |
| E11 | $C_{17}H_{35}$- | 63-64° | 2.20 2.55 |

These compound numbers will be referred to in the description below.

2) Preparation A

Preparation Example A1 (Tablet and giant pill)

20 g of Compound No.A3 and 78 g of calcium carbonate were milled and mixed well in a ball mill. 2 g of magnesium stearate was further added thereto, and the mixture was further milled and mixed. The mixture was processed by a tabletting machine to produce tablets each of 1 g (200 mg as effective ingredient). This tablet was administered orally in various doses from 0.2 to 250 g per day depending on the body weight of the animal. By varying the shapes of the mortar and the pestle of the tabletting machine, giant pills can be produced according to the same procedure as described above.

Preparation Example A2 (Liquid preparation)

A liquid preparation was prepared by mixing with stirring 50 g of a compound of Compound No.A1, 49 g of ethanol and 1 g of polyvinyl alcohol. This liquid preparation was mixed under stirring into the drinking water for an animal to a concentration of 1% to be suspended in the drinking water, and the resultant suspension was administered to the subject animal.

Preparation Example A3 (Feed mixture)

A solution of 20 g of a compound of Compound No.A12 in 20 ml of ethanol was further added to 80 g of lactose and mixed therewith with stirring, and thereafter ethanol was evaporated under reduced pressure to be completely removed. The mixture was mixed into feed for animal in an

amount of 50 g to 2,500 g per ton of the feed depending on the infected quantity of parasites in the animal. In this case, the proportion of the present compound in the feed was 0.001 to 0.05% by weight.

Preparation Example A4 (Capsule)

20 g of Compound No.A9 and 78 g of calcium carbonate were milled and mixed well in a ball mill. 2 g of magnesium stearate was further added thereto, and the mixture was further milled and mixed. The powder of this mixture was sub-divided into quantities each of 0.5 g and encapsulated in hard gelatin capsules. In each capsule was contained 100 mg of the present compound. One to 10 capsules per 10 Kg of body weight were administered to an animal depending on the body weight of the animal. In this case, the dose of administration of the present compound to the animal was 10 mg to 100 mg per Kg of body weight.

Preparation Example A5 (preparation for injection)

To 20 g of Compound No.A10 were added 1 g of sodium carboxymethyl cellulose, 0.2 g of sodium citrate and distilled warter for injection to prepare a mixture of a total quantity of 100 ml, which was thoroughly mixed with stirring, to prepare a suspension for injection. These preparations for injection were injected subcutaneously, intramuscularly, intraperitoneally or intravaneously in amounts varied from 0.01 ml to 50 ml per day depending on the body weight of the animal.

3) Anthelmintic effect A

Test Example A1

(1) Experimental method

Chickens infected artificially with fowl roundworm (Ascaridia galli) were employed in groups each consisting of three chickens. Fecal matter testing was conducted before administration of medicine to confirm infection with the worm. The quantity of the compound provided for testing was 10 mg and 20 mg per Kg of body weight, which was orally administered each in a gelatin capsule to the chicken. The number of worm bodies discharged within 48 hours after administration of medicine was counted, the chicken was killed after 48 hours and the number of worm bodies remaining in the enteron was counted to determine the anthelmintic percentage, wherefrom the antihelmintic effect was judged.

(2) Experimental results

As shown in Table A2, marked anthelmintic effect was observed in every compound of N-propargyl-N-acylhydroxylamine derivatives of the present invention.

The representation of anthelmintic effect is given according to the following ranks.

Anthelmintic percentage (%) =

$$\frac{\text{Discharged worm bodies}}{\text{Discharged worm bodies + Remaining worm bodies}} \times 100$$

| Anthelmintic percentage | Representation of anthelmintic effect |
|---|---|
| 100% | +++ |
| 80 - 90% | ++ |
| 50 - 79% | + |
| 1 - 49% | ± |
| 0% | - |

Table A2

| Compound NO. \ Dose (mg) \ Chicken No. \ Anthelmintic effect | | Anthelmintic effect after 48 hrs. | | |
|---|---|---|---|---|
| | | No.1 | No.2 | No.3 |
| A1 | 10 | +++ | +++ | +++ |
| | 20 | +++ | +++ | +++ |
| A2 | 10 | +++ | +++ | +++ |
| | 20 | +++ | +++ | +++ |
| A3 | 10 | ++ | +++ | +++ |
| | 20 | +++ | +++ | +++ |
| A4 | 10 | ++ | ++ | ++ |
| | 20 | +++ | +++ | +++ |
| A5 | 10 | +++ | +++ | +++ |
| | 20 | +++ | +++ | +++ |
| A6 | 10 | +++ | +++ | +++ |
| | 20 | +++ | +++ | +++ |
| A7 | 10 | +++ | ++ | ++ |
| | 20 | +++ | +++ | +++ |
| A8 | 10 | +++ | ++ | ++ |
| | 20 | +++ | +++ | +++ |
| A9 | 10 | ++ | ++ | ++ |
| | 20 | +++ | +++ | +++ |
| A10 | 10 | +++ | +++ | +++ |
| | 20 | +++ | +++ | +++ |
| A11 | 10 | +++ | +++ | +++ |
| | 20 | +++ | +++ | +++ |
| A12 | 10 | +++ | +++ | +++ |
| | 20 | +++ | +++ | +++ |

(continued)

| | | | | |
|---|---|---|---|---|
| A13 | 10 | +++ | +++ | +++ |
| | 20 | +++ | +++ | +++ |
| Control | 0 | - | - | - |
| | 0 | - | - | - |

Test Example A2

(1) Experimental method

Chickens artificially infected with fowl roundworm (As. galli) were employed in groups each of two chickens. The compound provided for the test was adsorbed on lactose as a solution dissolved in a small amount of ethyl alcohol and added to a chicken feed at levels of 10 ppm and 50 ppm.

Fecal matter testing was conducted before initiation of the test and on the 7th, 14th and 21st day after initiation of the test. Reduction in worm egg number was examined by calculating E.P.G. (worm eggs per 1 g of fecal matter), and the number of discharged worm bodies and the number of worm bodies remaining in the enteron when the chicken was killed on 21st day were counted to determine the anthelmintic percentage, from which the anthelmintic effect was judged.

(2) Experimental results

As shown in Table A3, marked anthelmintic effect was observed in every compound of N-propargyl-N-acylhydroxylamine derivatives of the present invention.

The representation of anthelmintic effect is the same as described above.

Table A3

| Compound No. | Amount added (ppm) * | Chicken No. | Change of E.P.G. Before administration | 7th day | 14th day | 21st day | Anthelmintic effect |
|---|---|---|---|---|---|---|---|
| A1 | 10 | 1<br>2 | 5,600<br>7,900 | 3,100<br>3,000 | 1,000<br>800 | 100<br>300 | ++<br>++ |
| | 50 | 3<br>4 | 10,700<br>14,300 | 4,300<br>100 | 0<br>100 | 0<br>0 | +++<br>+++ |
| A2 | 10 | 5<br>6 | 21,400<br>9,300 | 11,000<br>5,200 | 13,600<br>1,000 | 1,000<br>100 | ++<br>++ |
| | 50 | 7<br>8 | 27,600<br>17,400 | 3,300<br>100 | 0<br>0 | 0<br>0 | +++<br>+++ |
| A3 | 10 | 9<br>10 | 5,800<br>16,700 | 1,200<br>2,400 | 600<br>2,600 | 0<br>700 | +++<br>++ |
| | 50 | 11<br>12 | 29,300<br>36,000 | 700<br>1,300 | 0<br>100 | 0<br>0 | +++<br>+++ |
| A4 | 10 | 13<br>14 | 8,200<br>11,600 | 5,300<br>3,200 | 3,400<br>1,300 | 1,300<br>0 | ++<br>+++ |
| | 50 | 15<br>16 | 19,400<br>7,400 | 1,300<br>300 | 500<br>0 | 0<br>0 | +++<br>+++ |
| A5 | 10 | 17<br>18 | 9,500<br>10,600 | 8,000<br>4,300 | 3,300<br>1,000 | 1,300<br>200 | ++<br>++ |
| | 50 | 19<br>20 | 4,700<br>14,600 | 700<br>2,800 | 100<br>100 | 0<br>0 | +++<br>+++ |
| A6 | 10 | 21<br>22 | 15,600<br>9,300 | 1,800<br>5,400 | 500<br>1,000 | 0<br>300 | +++<br>++ |
| | 50 | 23<br>24 | 14,600<br>13,000 | 1,200<br>300 | 100<br>0 | 0<br>0 | +++<br>+++ |
| A7 | 10 | 25<br>26 | 10,600<br>8,900 | 7,600<br>3,100 | 2,000<br>1,300 | 500<br>200 | ++<br>++ |
| | 50 | 27<br>28 | 26,800<br>19,300 | 2,700<br>300 | 500<br>0 | 0<br>0 | +++<br>+++ |
| A8 | 10 | 29<br>30 | 14,500<br>10,600 | 5,600<br>1,800 | 900<br>2,100 | 0<br>1,000 | +++<br>++ |
| | 50 | 31<br>32 | 29,100<br>21,000 | 1,200<br>800 | 200<br>0 | 0<br>0 | +++<br>+++ |

(cotinued)

| | | | | | | | |
|---|---|---|---|---|---|---|---|
| A9 | 10 | 33<br>34 | 11,800<br>9,700 | 2,300<br>5,300 | 500<br>2,600 | 0<br>1,200 | +++<br>++ |
| | 50 | 35<br>36 | 31,000<br>19,400 | 10,300<br>7,400 | 2,200<br>700 | 0<br>0 | +++<br>+++ |
| A10 | 10 | 37<br>38 | 13,200<br>9,800 | 2,100<br>5,200 | 1,100<br>2,600 | 100<br>800 | ++<br>++ |
| | 50 | 39<br>40 | 6,500<br>11,400 | 700<br>1,300 | 0<br>500 | 0<br>0 | +++<br>+++ |
| A11 | 10 | 41<br>42 | 17,200<br>3,800 | 10,600<br>3,000 | 3,400<br>1,200 | 1,300<br>300 | ++<br>++ |
| | 50 | 43<br>44 | 17,300<br>8,800 | 300<br>3,100 | 0<br>100 | 0<br>0 | +++<br>+++ |
| A12 | 10 | 45<br>46 | 7,600<br>8,400 | 2,100<br>1,900 | 500<br>900 | 100<br>500 | ++<br>++ |
| | 50 | 47<br>48 | 9,300<br>13,700 | 0<br>1,200 | 0<br>0 | 0<br>0 | +++<br>+++ |
| A13 | 10 | 49<br>50 | 19,800<br>7,600 | 10,300<br>5,800 | 300<br>1,000 | 100<br>0 | ++<br>+++ |
| | 50 | 51<br>52 | 33,400<br>25,600 | 3,600<br>5,300 | 700<br>1,000 | 0<br>0 | +++<br>+++ |
| Con-trol | 0<br>0 | 53<br>54 | 18,200<br>7,400 | 20,600<br>13,800 | 15,300<br>15,600 | 21,300<br>13,200 | -<br>- |

* Parts by million

Test Example A3

(1) Experimental method

Three dogs with mixed infections by canine whipworm (Trichuris vulpis), canine hookworm (Ancylostoma caninum) and canine roundworm (Toxocara canis) (naturally infected dogs) were employed in a group of two and a group of one.

The compound provided for the test was Compound No.A1, and 20 mg/Kg thereof was placed in a gelatin capsule per 1 Kg of body weight, and administered orally to the two dogs. As control, only a gelatin capsule was administered orally to the one dog.

Before administration and on the 7th day after administration, fecal matter testing was conducted to calculate the E.P.G., and the anthelmintic effect was judged by the changing of the E.P.G. to negative.

(2) Experimental results

As shown in Table A4, marked anthelmintic effect was observed with one administration of 20 mg/Kg of the Compound A1.

Table A4

Test of anthelmintic effect against canine whipworm, canine hookworm and canine roundworm

| Dose (mg/Kg) | Body weight of dog (Kg) | Kind of parasite | Change of E.P.G. | |
|---|---|---|---|---|
| | | | Before administration | 7th day |
| 20 | 6.2 | Trichuris vulpis | 3,200 | 0 |
| | | Ancylostoma caninum | 4,500 | 0 |
| | | Toxocara canis | 5,100 | 0 |
| 20 | 10.5 | Trichuris vulpis | 2,700 | 0 |
| | | Ancylostoma caninum | 7,800 | 0 |
| | | Toxocara canis | 2,300 | 0 |
| Control | 8.4 | Trichuris vulpis | 2,200 | 1,900 |
| | | Ancylostoma caninum | 7,500 | 8,100 |
| | | Toxocara canis | 6,500 | 7,000 |

Test Example A4

(1) Experimental method

Three dogs infected with canine strongyloides (naturally infected dogs) were employed by grouping them into a

group of two and a group of one.

The compound provided for the test was Compound No.A3, and 10 mg/Kg thereof was placed in a gelatin capsule per 1 Kg of body weight, and administered orally to the two dogs. As control, only a gelatin capsule was administered orally to the one dog.

Before administration and on the 7th day after administration, fecal matter testing was conducted to calculate the E.P.G., and the anthelmintic effect was judged by the changing of the E.P.G. to negative.

(2) Experimental results

As shown in Table A5, marked anthelmintic effect was observed with one administration of 10 mg/Kg of the compound A1.

Table A5

Anthelmintic test against canine strongyloides

| Dose (mg/Kg) | Body weight of dog (Kg) | Kind of parasite | Change of E.P.G. | |
|---|---|---|---|---|
| | | | Before administration | 7th day |
| 10 | 11.5 | Strongyloides stercoralis | 1,900 | 0 |
| 10 | 10.5 | " | 2,700 | 0 |
| Control | 8.4 | " | 600 | 500 |

Test Example A5

(1) Experimental method

Six cats were employed. The cats judged to be healthy after destruction of various parasites in their digestive organs during quarantine of 2 weeks or longer were employed as experimental cats. Each experimental cat was infected orally with 5 plerocercoids of Diphyllobothrium erinacei collected from beneath the skin of a snake (Rhabdophis) and placed in a gelatin capsule. After infection, fecal matter was tested according to the direct smear method on the 10th day and every day thereafter. Infection was judged to be established by detection of worm eggs. The anthelmintic test was performed 15 days after plerocercoid infection.

The compounds provided for the test were Compound Nos. A3 and A12, and 50 mg thereof per Kg of body weight was placed in a gelatin capsule and administered orally to each of two cats once per day continuously for 5 days. As control, to each of two of the cats, only one gelatin capsule per day was administered orally for 5 days.

Ten days after administration, all the cats were sacrificed by anesthesia with pentobarbital for autopsy, and judgement was made by examination of the worm bodies remaining in the enteron.

(2) Experimental results

As shown in Table A7, marked anthelmintic effects were observed in the case of 5 continuous administrations

of 50 mg/Kg of the respective compounds of Compound Nos. A3 and A12.

Table A7

Test of anthelmintic effect against Diphyllobothrium erinacei (tapeworm)

| Compound No. | Dose mg/Kg x times | Body weight of experimental cat (Kg) | Worm egg detection before administration | Worm egg detection on 10th day after administration | Number of worm bodies remaining in enteron |
|---|---|---|---|---|---|
| A3 | 50 x 5 times | 2.1 | + | - | 0 |
| | " | 1.8 | + | - | 0 |
| A12 | " | 2.0 | + | - | 0 |
| | " | 2.3 | + | - | 0 |
| Control | Only gelatin capsule x 5 times | 1.5 | + | + | 5 |
| | | 1.3 | + | + | 3 |

In the Table, + indicates detection of Diphyllobothrium's egg, and - no detection.

Test Example A6

Three cows infected with stomach worm (naturally infected cows) were employed by grouping them into a group of two and a group of one.

Compound No.A3 was used for the test, and 15 mg thereof was placed in a gelatin capsule per 1 Kg of body weight and administered orally to the two cows. As control,

only gelatin capsules were administered orally to the one cow.

Before administration and on the 7th day after administration, fecal matter testing was conducted to calculate the E.P.G., and the anthelmintic effect was judged by the changing of the E.P.G. to negative.

(2) Experimental results

As shown in Table A8, marked anthelmintic effect was observed with one administration of 15 mg/Kg of the compound A3.

Table A8

Anthelmintic test against cow stomach worm

| Dose (mg/Kg) | Body weight of cow (Kg) | Kind of parasite | Change of E.P.G. | |
|---|---|---|---|---|
| | | | Before administration | 7th day |
| 15 | 530 | Haemonchus controtus | 1,700 | 0 |
| 15 | 510 | " | 1,500 | 0 |
| Control | 450 | " | 1,200 | 1,400 |

Test Example A7

Two sheep infected with Trichostrongylidae (naturally infected sheep) were employed.

The compound provided for the test was Compound No. A1, 15 mg of which was placed in a gelatin capsule

per 1 Kg of body weight and administered orally to one of the sheep. As a control, only gelatin capsules were administered orally to the other sheep.

Before administration and on the 7th day after administration, fecal matter testing was conducted to calculate the E.P.G., and the anthelmintic effect was judged by the changing of the E.P.G. to negative.

(2) Experimental results

As shown in Table A9, marked anthelmintic effect was observed with one administration of 15 mg/Kg of the compound A1.

Table A9

Anthelmintic test against sheep Trichostrongyldae

| Dose (mg/Kg) | Body weight of sheep (Kg) | Kind of parasite | Change of E.P.G. | |
|---|---|---|---|---|
| | | | Before administration | 7th day |
| 15 | 35 | Tricostrongylus colubriformis | 1,600 | 0 |
| Control | 40 | " | 1,200 | 1,400 |

Test Example A8

Six pigs infected with swine Oesophagostomum (naturally infected pigs) were employed by grouping them into groups each of two pigs.

The compounds provided for the test were Compound Nos. A3 and A12, 20 mg of each of which was placed in a gelatin capsule per 1 Kg of body weight and administered orally to each of the two pigs. As a control, only gelatin capsules were administered orally to the two pigs.

Before administration and on the 7th day after administration, fecal matter testing was conducted to calculate the E.P.G., and the anthelmintic effect was judged by the changing of the E.P.G. to negative.

(2) Experimental results

As shown in Table A10, marked anthelmintic effect was observed with one administration of 20 mg/Kg of the compound A3 and A12, respectively.

Table A10

Test of anthelmintic effect
against swine Oesophagostomum

| Compound No. | Dose (mg/Kg) | Body weight of pig (Kg) | Kind of parasite | Change of E.P.G. | |
|---|---|---|---|---|---|
| | | | | Before administration | 7th day |
| A3 | 20 | 80 | Oesophagostomum dentatum | 1,600 | 0 |
| | 20 | 91 | " | 1,000 | 0 |
| A12 | 20 | 87 | " | 2,100 | 0 |
| | 20 | 88 | " | 1,500 | 0 |
| Control | Only gelatin capsule | 75 | " | 1,200 | 1,500 |
| | | 70 | " | 900 | 1,000 |

Test Example A9

(1) Experimental method

Six pigs infected with swine roundworm and swine whipworm (naturally infected pigs) were employed in groups each of two pigs.

The compounds provided for the test were Compound Nos. Al and Al0, 20 mg each of which was placed in a gelatin capsule per 1 Kg of body weight, and administered orally to each of the two pigs. As a control, only gelatin capsules were administered orally to the two pigs.

Before administration and on the 7th day after administration, fecal matter testing was conducted to calculate the E.P.G., and the anthelmintic effect was judged by the changing of the E.P.G. to negative.

(2) Experimental results

As shown in Table All, marked anthelmintic effect was observed with one administration of 20 mg/Kg of the Compound Al and Compound Al0, respectively.

Table All

Test of anthelmintic effect

against swine roundworm and swine whipworm

| Compound No. | Dose (mg/Kg) | Body weight of pig (Kg) | Kind of parasite | Change of E.P.G. | |
|---|---|---|---|---|---|
| | | | | Before administration | 7th day |
| A1 | 20 | 65 | Ascaris suum | 3,600 | 0 |
| | | | Trichuris suis | 1,300 | 0 |
| | 20 | 63 | Ascaris suum | 1,000 | 0 |
| | | | Trichuris suis | 2,100 | 0 |
| A10 | 20 | 60 | Ascaris suum | 4,300 | 0 |
| | | | Trichuris suis | 3,800 | 0 |
| | 20 | 68 | Ascaris suum | 900 | 0 |
| | | | Trichuris suis | 1,100 | 0 |
| Control | Only gelatin capsule | 55 | Ascaris suum | 1,200 | 800 |
| | | | Trichuris suis | 2,100 | 2,000 |
| | Only gelatin capsule | 50 | Ascaris suum | 300 | 200 |
| | | | Trichuris suis | 500 | 700 |

Test Example A10

(1) Experimental method

Eight dogs infected with canine filaria; Dirofilaria immitis (naturally infected dogs) were employed in groups each of two dogs.

The compounds provided for tests were Compound Nos. A1, A3 and A12. 20 mg of each was administered by subcutaneous injection per Kg of body weight to each of the two dogs as a suspension in 1% "Tween 80" solution once per day for three days. As control, 3 ml of the 1% "Tween 80" solution was subcutaneously injected into two dogs once per day for three days.

Before administration and on the 10th day after administration, 20 µl of earlobe blood of each dog was sampled and examined to determine the change in microfilaria (mf) according to the concentrated smear color method, and judgement on the effect on microfilaria (mf) was made. Judgement relating to adult filaria was made on the 10th day after the last administration by killing the dogs and carrying out autopsis.

(2) Experimental results

As shown in Table 12, marked anthelmintic effect was observed against microfilaria (mf) in each of the compounds of Compound Nos. A1, A3 and A12 with administration of 20 mg/Kg repeated three times, while about 50% or more of anthelmintic effect was observed against adult filaria.

Table A12

Test of anthelmintic effect against canine filaria

| Compound No. | Dose x times mg/Kg x times | Body weight of dog (Kg) | Number of microfilaria/20 μl | | Effect on adult filaria | |
|---|---|---|---|---|---|---|
| | | | Before administration | On 10th day after the last administration | Number of worms killed/Total parasites | Percent of worms killed |
| A1 | 20 x 3 | 10.5 | 21 | 0 | 7/12 | 58 |
| | 20 x 3 | 7.7 | 16 | 0 | 5/ 7 | 71 |
| A3 | 20 x 3 | 8.6 | 17 | 0 | 13/18 | 72 |
| | 20 x 3 | 11.4 | 10 | 0 | 4/ 8 | 50 |
| A12 | 20 x 3 | 9.6 | 36 | 0 | 13/21 | 62 |
| | 20 x 3 | 9.0 | 27 | 0 | 15/26 | 58 |
| Control | 1% [Tween80] | 7.1 | 15 | 11 | 0/ 9 | 0 |
| | 3ml x 3 | 8.4 | 18 | 21 | 0/13 | 0 |

Test Example All

(1) Experimental method

Twenty-eight (28) rats were employed in fourteen groups each of two rats, each rat being infected with 5 metacercariae of Liver fluke; Fasciola hepatica (F. hepatica) and confirmed to have worm eggs on the 75th day after infection according to fecal matter testing.

The compounds provided for the tests were Compound Nos. Al through Al3, 50 mg of each of which per Kg of body weight was suspended in 5% gum arabic solution and administered orally once per day continuously for 5 days. As control, only one ml of 5% gum arabic solution was administered orally once per day continuously for 5 days.

Judgement of the effect was made by the presence or absence of the worm gees in the fecal matter on the 21st day after the last administration and by examination according to autopsy as to whether the worm bodies were dead or alive in the liver.

(2) Experimental results

As shown in Table Al3, marked antihelmintic effect was observed in each compound of the N-propargyl-N-aceylhydroxylamine derivatives of the present invention.

Table A13

Test of anthelmintic effect against rat infected experimentally with liver fluke (F. hepatica)

| Compound No. | Dose x times mg/Kg x times | Presence or absence of fluke egg in fecal matter | | Condition of fluke body |
|---|---|---|---|---|
| | | Before administration | On 21st day after the last administration | |
| A1 | 50 x 5 | + | - | dead |
| | 50 x 5 | + | - | dead |
| A2 | 50 x 5 | + | - | dead |
| | 50 x 5 | + | - | dead |
| A3 | 50 x 5 | + | - | dead |
| | 50 x 5 | + | - | dead |
| A4 | 50 x 5 | + | - | dead |
| | 50 x 5 | + | - | dead |
| A5 | 50 x 5 | + | - | dead |
| | 50 x 5 | + | - | dead |
| A6 | 50 x 5 | + | - | dead |
| | 50 x 5 | + | - | denatured |
| A7 | 50 x 5 | + | - | dead |
| | 50 x 5 | + | - | dead |
| A8 | 50 x 5 | + | - | dead |
| | 50 x 5 | + | - | dead |
| A9 | 50 x 5 | + | - | dead |
| | 50 x 5 | + | - | dead |
| A10 | 50 x 5 | + | - | dead |
| | 50 x 5 | + | - | dead |
| A11 | 50 x 5 | + | - | dead |
| | 50 x 5 | + | - | dead |
| A12 | 50 x 5 | + | - | dead |
| | 50 x 5 | + | - | dead |

(continued)

| | | | | |
|---|---|---|---|---|
| A13 | 50 x 5 | + | - | denatured |
| | 50 x 5 | + | - | dead |
| Control | 5% gum arabic solution 1 ml x 5 | + | + | alive |
| | 5% gum arabic solution 1 ml x 5 | + | + | alive |

Note: + indicates detection of fluke egg, and

- indicates no detection.

"Denatured" indicates that fluke body is alive but a part of fluke body has changed.

2) Preparation B

Preparation Example (Tablet and giant pill)

Compound No.B1 (20 g) and 78 g of calcium carbonate were milled and mixed well in a ball mill. 2 g of magnesium stearate was further added thereto, and the mixture was further crushed and mixed. The mixture was fed into a tabletting machine to produce tablets each of 1g (200 mg as effective ingredient). This tablet was administered orally at various doses from 0.2 to 250 g per day depending on the body weight of an animal. By varying the shapes of the mortar and the pestle of the tabletting machine, giant pills can be produced according to the same procedure as described above.

Preparation Example B2 (Liquid preparation)

A liquid preparation was prepared by mixing with stirring 50 g of Compound No. B8, 49 g of ethanol and 1 g of polyvinyl alcohol. This liquid preparation was mixed with stirring into the drinking water for the animal to a concentration of 1% to be suspended in the drinking water, and the resultant suspension was administered to the subject animal.

Preparation Example B3 (Feed mixture)

A solution of 20 g of the Compound No.B19 in 20 ml of ethanol was further added to 80 g of lactose and mixed therewith with stirring, and thereafter the ethanol was evaporated off under reduced pressure to be completely

removed. The mixture was mixed into feed for the animal in an amount of 50 g to 2,500 g per ton of the feed depending on the quantity of infecting parasites in the animal. In this case, the proportion of the present compound in feed was 0.001 to 0.05% by weight.

Preparation Example B4 (Capsule)

Compound No.B20 (20 g) and 78 g of calcium carbonate were milled and mixed well in a ball mill. 2 g of magnesium stearate was further added thereto, and the mixture was further milled and mixed. The powder of this mixture was sub-divided into divisions each of 0.5 g which were then placed in hard gelatin capsules. In each capsule was contained 100 mg of the present compound. One to 10 capsules per 10 Kg of body weight were administered to an animal depending on its body weight. In this case, the dose of administration of the present compound to the animal was 10 mg to 100 mg per Kg of body weight.

Preparation Example B5 (Preparation for injection)

To 20 g of Compound No. B1 were addded 1 g of sodium carboxymethyl cellulose, 0.2 g of sodium citrate and distilled water for injection to make up a total quantity of 100 ml, and the mixture was thoroughly mixed with stirring to prepare a suspension for injection. These preparations for injection were injected subcutaneously, intramuscularly, intraperitoneally or intravaneously in amounts varied from 0.01 ml to 50 ml per day depending on the body weight of the animal.

3) Anthelmintic effect B

Test Example B1

(1) Experimental method

The same as that in Test Example A1.

(2) Experimental results

As shown in Table B2, marked anthelmintic effect was observed in every compound of N-alkenyl-N-acylhydroxylamine derivatives of the formula (I-2).

The representation of anthelmintic effect is the same as that in Test Example A1.

Table B2

| Compound No. / Dose (mg/Kg) / Chicken No. (Anthelmintic effect) | | Anthelmintic effect after 48 hours | | |
|---|---|---|---|---|
| Com-pound No. | Dose (mg/Kg) | No.1 | No.2 | No.3 |
| B1 | 30 | +++ | +++ | +++ |
| | 50 | +++ | +++ | +++ |
| B2 | 30 | +++ | +++ | +++ |
| | 50 | +++ | +++ | +++ |
| B3 | 30 | +++ | +++ | +++ |
| | 50 | +++ | +++ | +++ |
| B4 | 30 | +++ | +++ | +++ |
| | 50 | +++ | +++ | +++ |
| B5 | 30 | +++ | +++ | +++ |
| | 50 | +++ | +++ | +++ |
| B6 | 30 | +++ | +++ | +++ |
| | 50 | +++ | +++ | +++ |
| B7 | 30 | +++ | +++ | +++ |
| | 50 | +++ | +++ | +++ |
| B8 | 30 | +++ | +++ | +++ |
| | 50 | +++ | +++ | +++ |
| B9 | 30 | +++ | +++ | +++ |
| | 50 | +++ | +++ | +++ |
| B10 | 30 | +++ | +++ | +++ |
| | 50 | +++ | +++ | +++ |
| B11 | 30 | +++ | +++ | +++ |
| | 50 | +++ | +++ | +++ |
| B12 | 30 | +++ | +++ | +++ |
| | 50 | +++ | +++ | +++ |
| B13 | 30 | +++ | +++ | +++ |
| | 50 | +++ | +++ | +++ |

(continued)

| | | | | |
|---|---|---|---|---|
| B14 | 30<br>50 | +++<br>+++ | +++<br>+++ | +++<br>+++ |
| B15 | 30<br>50 | +++<br>+++ | +++<br>+++ | +++<br>+++ |
| B16 | 30<br>50 | +++<br>+++ | +++<br>+++ | +++<br>+++ |
| B17 | 30<br>50 | +++<br>+++ | +++<br>+++ | +++<br>+++ |
| B18 | 30<br>50 | +++<br>+++ | +++<br>+++ | +++<br>+++ |
| B19 | 30<br>50 | +++<br>+++ | +++<br>+++ | +++<br>+++ |
| B20 | 30<br>50 | +++<br>+++ | +++<br>+++ | +++<br>+++ |
| Control | 0 | - | - | - |

Test Example B2

(1) Experimental method

The same as that in Test Example A2, except that the compound provided for the test was added to a chicken feed at levels of 20 ppm and 50 ppm.

(2) Experimental results

As shown in Table B3, marked anthelmintic effect was observed in every compound of N-alkenyl-N-acylhydroxylamine derivatives of the formula (I-2).

The representation of anthelmintic effect was the same as that described above.

Table B3

| Compound No. | Amount added (ppm) | Chicken No. | Change of E.P.G. Before administration | 7th day | 14th day | 21st day | Anthelmintic effect |
|---|---|---|---|---|---|---|---|
| B1 | 20 | 1 | 9,500 | 8,000 | 3,300 | 1,300 | ++ |
| | | 2 | 11,600 | 3,200 | 1,300 | 0 | +++ |
| | 50 | 3 | 29,300 | 700 | 0 | 0 | +++ |
| | | 4 | 17,400 | 100 | 0 | 0 | +++ |
| B2 | 20 | 1 | 8,200 | 5,300 | 3,400 | 1,300 | ++ |
| | | 2 | 16,700 | 2,400 | 2,600 | 700 | ++ |
| | 50 | 3 | 29,300 | 1,300 | 500 | 0 | +++ |
| | | 4 | 36,000 | 2,400 | 100 | 0 | +++ |
| B3 | 20 | 1 | 5,600 | 3.100 | 1,000 | 100 | ++ |
| | | 2 | 7,900 | 3,000 | 800 | 300 | ++ |
| | 50 | 3 | 10,700 | 4,300 | 0 | 0 | +++ |
| | | 4 | 14,300 | 100 | 100 | 0 | +++ |
| B4 | 20 | 1 | 5,800 | 1,200 | 600 | 0 | +++ |
| | | 2 | 16,700 | 2,400 | 2,600 | 700 | ++ |
| | 50 | 3 | 19,400 | 1,300 | 500 | 0 | +++ |
| | | 4 | 7,400 | 300 | 0 | 0 | +++ |
| B5 | 20 | 1 | 13,200 | 2,100 | 1,100 | 100 | ++ |
| | | 2 | 9,800 | 5,200 | 2,600 | 800 | ++ |
| | 50 | 3 | 31,000 | 10,300 | 2,200 | 0 | +++ |
| | | 4 | 19,400 | 7,400 | 700 | 0 | +++ |
| B6 | 20 | 1 | 21,400 | 11,000 | 13,600 | 1,000 | ++ |
| | | 2 | 8,200 | 5,300 | 500 | 0 | +++ |
| | 50 | 3 | 10,700 | 5,200 | 100 | 0 | +++ |
| | | 4 | 5,600 | 4,300 | 0 | 0 | +++ |

(continued)

| | | | | | | | |
|---|---|---|---|---|---|---|---|
| B7 | 20 | 1 | 13,200 | 2,100 | 1,100 | 100 | ++ |
| | | 2 | 9,800 | 5,200 | 2,600 | 800 | ++ |
| | 50 | 3 | 6,500 | 700 | 0 | 0 | +++ |
| | | 4 | 11,400 | 1,300 | 500 | 0 | +++ |
| B8 | 20 | 1 | 15,600 | 1,800 | 500 | 0 | +++ |
| | | 2 | 9,300 | 5,400 | 1,000 | 300 | ++ |
| | 50 | 3 | 14,600 | 1,200 | 100 | 0 | +++ |
| | | 4 | 13,000 | 300 | 0 | 0 | +++ |
| B9 | 20 | 1 | 11,800 | 2,300 | 500 | 0 | +++ |
| | | 2 | 9,700 | 5,300 | 2,600 | 1,200 | ++ |
| | 50 | 3 | 31,000 | 10,300 | 2,200 | 0 | +++ |
| | | 4 | 19,400 | 7,400 | 700 | 0 | +++ |
| B10 | 20 | 1 | 10,600 | 7,600 | 2,000 | 500 | ++ |
| | | 2 | 8,900 | 3,100 | 1,300 | 200 | ++ |
| | 50 | 3 | 26,800 | 2,700 | 500 | 0 | +++ |
| | | 4 | 19,300 | 300 | 0 | 0 | +++ |
| B11 | 20 | 1 | 14,500 | 5,600 | 900 | 0 | +++ |
| | | 2 | 10,200 | 1,600 | 1,900 | 600 | ++ |
| | 50 | 3 | 28,400 | 1,600 | 100 | 0 | +++ |
| | | 4 | 21,300 | 800 | 0 | 0 | +++ |
| B12 | 20 | 1 | 13,500 | 7,400 | 1,600 | 100 | ++ |
| | | 2 | 9,800 | 1,300 | 1,500 | 300 | ++ |
| | 50 | 3 | 28,600 | 3,400 | 400 | 0 | +++ |
| | | 4 | 18,400 | 500 | 0 | 0 | +++ |
| B13 | 20 | 1 | 19,800 | 10,300 | 300 | 100 | ++ |
| | | 2 | 7,600 | 5,800 | 1,000 | 0 | +++ |
| | 50 | 3 | 33,400 | 3,600 | 700 | 0 | +++ |
| | | 4 | 25,600 | 5,300 | 1,000 | 0 | +++ |
| B14 | 20 | 1 | 17,200 | 10,600 | 3,400 | 1,300 | ++ |
| | | 2 | 3,800 | 3,000 | 1,200 | 100 | ++ |
| | 50 | 3 | 17,300 | 900 | 0 | 0 | +++ |
| | | 4 | 8,800 | 3,100 | 100 | 0 | +++ |

(continued)

| | | | | | | | |
|---|---|---|---|---|---|---|---|
| B15 | 20 | 1 | 7,600 | 2,100 | 500 | 100 | ++ |
| | | 2 | 8,400 | 1,900 | 900 | 500 | ++ |
| | 50 | 3 | 9,300 | 0 | 0 | 0 | +++ |
| | | 4 | 13,700 | 1,200 | 0 | 0 | +++ |
| B16 | 20 | 1 | 15,600 | 1,500 | 200 | 0 | +++ |
| | | 2 | 8,300 | 4,400 | 800 | 200 | ++ |
| | 50 | 3 | 15,300 | 1,300 | 100 | 0 | +++ |
| | | 4 | 13,200 | 500 | 0 | 0 | +++ |
| B17 | 20 | 1 | 11,600 | 6,700 | 2,000 | 500 | ++ |
| | | 2 | 9,800 | 1,300 | 800 | 100 | ++ |
| | 50 | 3 | 28,600 | 2,700 | 500 | 0 | +++ |
| | | 4 | 13,900 | 300 | 0 | 0 | +++ |
| B18 | 20 | 1 | 15,400 | 6,500 | 200 | 0 | +++ |
| | | 2 | 16,000 | 1,800 | 1,200 | 800 | ++ |
| | 50 | 3 | 21,900 | 2.100 | 200 | 0 | +++ |
| | | 4 | 21,500 | 800 | 0 | 0 | +++ |
| B19 | 20 | 1 | 5,600 | 3,100 | 1,000 | 100 | ++ |
| | | 2 | 7,900 | 3,000 | 800 | 0 | +++ |
| | 50 | 3 | 11,700 | 3,400 | 0 | 0 | +++ |
| | | 4 | 14,300 | 100 | 100 | 0 | +++ |
| B20 | 20 | 1 | 21,400 | 8,900 | 1,000 | 0 | +++ |
| | | 2 | 9,300 | 5,200 | 1,000 | 100 | ++ |
| | 50 | 3 | 27,600 | 3,300 | 0 | 0 | +++ |
| | | 4 | 17,400 | 100 | 0 | 0 | +++ |
| Con-trol | 0 | 1 | 18,200 | 20,600 | 15,300 | 21,300 | - |
| | | 2 | 7,400 | 13,800 | 15,600 | 13,200 | - |

Test Example B3

(1) Experimental method

The same as that in test Example A3, but the compound provided for the test was Compound No.B1, and 30 mg/Kg thereof was placed in a gelatin capsule per 1 Kg of body weight and administered orally to the two dogs. As control, only gelatin capsules were administered orally to the one dog.

(2) Experimental results

As shown in Table B4, marked anthelmintic effect was observed with one administration of 30 mg/Kg of the Compound B1.

Table B4

Test of anthelmintic effect against canine whipworm, canine hookworm and canine roundworm

| Dose (mg/Kg) | Body weight of dog (Kg) | Kind of parasite | Change of E.P.G. | |
|---|---|---|---|---|
| | | | Before administ-ration | 7th day |
| 30 | 7.5 | Trichuris vulpis | 5,100 | 0 |
| | | Ancylostoma caninum | 4,500 | 0 |
| | | Toxocara canis | 6,100 | 0 |
| 30 | 10.5 | Trichuris vulpis | 2,700 | 0 |
| | | Ancylostoma caninum | 8,700 | 0 |
| | | Toxocara canis | 3,200 | 0 |
| Con-trol | 9.5 | Trichuris vulpis | 3,400 | 2,900 |
| | | Ancylostoma caninum | 5,700 | 6,200 |
| | | Toxocara canis | 5,600 | 6,100 |

Test Example B4

(1) Experimental method

The same as that in Test Example A4, but the compound provided for the test was Compound No.B8, and 20 mg/Kg thereof was placed in a gelatin capsule per 1 Kg of body weight and administered orally to the two dogs. As control, only gelatin capsules were administered orally to the one dog.

(2) Experimental results

As shown in Table B5, marked anthelmintic effect was observed with one administration of 20 mg/Kg of the Compound A1.

Table B5

Anthelmintic test against canine strongyloides

| Dose (mg/Kg) | Body weight of dog (Kg) | Kind of parasite | Change of E.P.G. | |
|---|---|---|---|---|
| | | | Before administration | 7th day |
| 20 | 9.5 | Strongyloides stercoralis | 5,300 | 0 |
| 20 | 7.0 | " | 2,600 | 0 |
| Control | 8.5 | " | 700 | 900 |

Test Example B5

(1) Experimental method

The same as that in Test Example A5, but the compounds provided for the test were Compound Nos. B1 and B19.

(2) Experimental results

As shown in Table B7, marked anthelmintic effects were observed with 5 continuous administrations each of 50 mg/Kg of the respective Compound Nos. B1 and B19.

Table B7

Test of anthelmintic effect against Diphyllobothrium erinacei

| Compound No. | Dose mg/Kg x times | Body weight of experimental cat (Kg) | Worm egg detection before administration | Worm egg detection on 10th day after administration | Number of worm bodies remaining in enteron |
|---|---|---|---|---|---|
| B1 | 50 x 5 | 2.3 | + | - | 0 |
| | " | 2.0 | + | - | 0 |
| B19 | " | 1.8 | + | - | 0 |
| | " | 2.1 | + | - | 0 |
| Contorl | Only gelatin capsule x 5 | 1.3 | + | + | 4 |
| | " | 1.6 | + | + | 2 |

In the Table, + indicates detection of Diphyllobothrium's egg, and - no detection.

Test Example B6

(1) Experimental Method

The same as that in Test Example A6, but the compound provided for the test was Compound No. B1, and 20 mg thereof was placed in a gelatin capsule per 1 Kg of body weight and administered orally to the two cows. As control, only gelatin capsules were administered orally to the one cow.

(2) Experimental results

As shown in Table B8, marked anthelmintic effect was observed with one administration of 20 mg/Kg of the compound B1.

Table B8

Test of anthelmintic effect against cow stomach worm

| Dose (mg/Kg) | Body weight of cow (Kg) | Kind of parasite | Change of E.P.G. | |
|---|---|---|---|---|
| | | | Before administration | 7th day |
| 20 | 410 | Haemonchus controtus | 2,500 | 0 |
| 20 | 470 | " | 3,300 | 0 |
| Control | 390 | " | 1,700 | 2,000 |

Test Example B7

(1) Experimental method

The same as that in Test Example A7, but the compound provided for the test was Compound No.B8, and 20 mg thereof was placed in a gelatin capsule per 1 Kg of body weight and administered orally to one of the sheep. As control, only gelatin capsules were administered orally to the other sheep.

(2) Experimental results

As shown in Table B9, marked anthelmintic effect was observed with one administration of 20 mg/Kg of the compound B8.

Table B9

Test of anthelmintic effect against sheep Trichostrongyldae

| Dose (mg/Kg) | Body weight of sheep (Kg) | Kind of parasite | Change of E.P.G. | |
|---|---|---|---|---|
| | | | Before administration | 7th day |
| 20 | 45 | Trichostrongylus colubriformis | 3,400 | 0 |
| Control | 47 | " | 1,200 | 1,500 |

Test Example B8

(1) Experimental method

The same as that in Test Example A8, except that the compounds provided for the test were Compound Nos. B1 and B20.

(2) Experimental results

As shown in Table B10, marked anthelmintic effect was observed with one administration of 20 mg/Kg of the Compound Nos. B1 and B20, respectively.

Table B10

Test of anthelmintic effect against swine Oesophagostomum

| Compound No. | Dose (mg/Kg) | Body weight of pig (Kg) | Kind of parasite | Change of E.P.G. | |
|---|---|---|---|---|---|
| | | | | Before administration | 7th day |
| B1 | 20 | 43 | Oesophagostomum dentatum | 1,200 | 0 |
| | 20 | 55 | " | 2,600 | 0 |
| B20 | 20 | 38 | " | 1,000 | 0 |
| | 20 | 41 | " | 1,600 | 0 |
| Control | Only gelatin capsule | 40 | " | 1,300 | 1,400 |
| | | 39 | " | 900 | 1,100 |

Test Example B9

(1) Experimental method

The same as that in Test Example A9, but the compounds provided for the test were Compound Nos. B5 and B19. 50 mg of each compound was placed in a gelatin capsule per 1 Kg of body weight and administered orally to each of the two pigs. As control, only gelatin capsules were administered orally to two pigs.

(2) Experimental results

As shown in Table B11, marked anthelmintic effect was observed with one administration of 50 mg/Kg of the Compound Nos. B5 and B19, respectively.

Table B11

Test of anthelmintic effect
against swine roundworm and swine whipworm

| Compound No. | Dose (mg/Kg) | Body weight of pig (Kg) | Kind of parasite | Chenge of E.P.G. | |
|---|---|---|---|---|---|
| | | | | Before administration | 7th day |
| B5 | 50 | 43 | Ascaris suum | 1,000 | 0 |
| | | | Trichuris suis | 2,100 | 0 |
| | 50 | 48 | Ascaris suum | 3,600 | 0 |
| | | | Trichuris suis | 1,300 | 0 |
| B19 | 50 | 51 | Ascaris suum | 4,400 | 0 |
| | | | Trichuris suis | 3,800 | 0 |
| | 50 | 42 | Ascaris suum | 1,300 | 0 |
| | | | Trichuris suis | 1,100 | 0 |
| Control | Only gelatin capsule | 43 | Ascaris suum | 1,900 | 2,100 |
| | | | Trichuris suis | 1,700 | 1,200 |
| | Only gelatin capsule | 50 | Ascaris suum | 400 | 600 |
| | | | Trichuris suis | 900 | 300 |

Test Example B10

(1) Experimental method

The same as that in Test Example A10, but the compounds provided for tests were Compound Nos. B1, B8, and B9. 30 mg of each compound was administered by subcutaneous injection per Kg of body weight to each of the two dogs as a suspension in 1% "Tween 80" solution once per day for three days. As control, 3 ml of the 1% "Tween 80" solution was subcutaneously injected into two dogs once per day for three days.

(2) Experimental results

As shown in Table B12, marked anthelmintic effect was observed against microfilaria (mf) in each of the compounds of Compound Nos. B1, B8 and B9 with administration of 30 mg/Kg repeated for three times, while about 50% or more of anthelmintic effect was observed against adult filaria.

Table B12

Test of anthelmintic effect against canine filaria

| Compound No. | Dose x Times mg/Kg x Times | Body weight of dog (Kg) | Number of microfilaria/20 µl | | Effect on adult filaria | |
|---|---|---|---|---|---|---|
| | | | Before administration | on 10th day after the last administration | Number of worms killed/Total parasite | Percent of worms killed |
| B1 | 30 x 3 | 11.4 | 17 | 0 | 8/15 | 58 |
| | 30 x 3 | 6.8 | 20 | 0 | 12/21 | 57 |
| B8 | 30 x 3 | 13.5 | 21 | 0 | 7/12 | 58 |
| | 30 x 3 | 8.5 | 11 | 0 | 13/21 | 62 |
| B9 | 30 x 3 | 7.5 | 26 | 0 | 5/7 | 71 |
| | 30 x 3 | 9.5 | 20 | 0 | 4/8 | 50 |
| Control | 1% [Tween 80] 3 ml x 3 | 7.0 | 18 | 24 | 0/31 | 0 |
| | | 7.3 | 13 | 17 | 0/9 | 0 |

Test Example B11

(1) Experimental method

The same as that in Test Example A11, but eight (8) rats were employed by grouping them into groups each of two rats, each rat being infected with 5 metacercariae of liver fluke and confirmed to have fluke eggs on the 75th day after infection according to fecal matter testing.

The compounds provided for the tests were Compound Nos. B1, B8 and B19.

(2) Experimental results

As shown in Table B13, marked antihelmintic effect was observed in each compound of Compound Nos. B1, B8 and B19 with five administrations in a dose of 50 mg/Kg each.

Table B13

Test of anthelmintic effect against rat infected experimentally with liver fluke (F. hepatica)

| Compound No. | Dose x Times<br><br>mg/Kg x Times | Presence or absence of fluke egg in fecal matter | | Condition of fluke body |
|---|---|---|---|---|
| | | Before administration | On 21st day after the last administration | |
| B1 | 50 x 5 | + | - | dead |
| | " | + | - | dead |
| B8 | " | + | - | dead |
| | " | + | - | dead |
| B19 | " | + | - | dead |
| | " | + | - | dead |
| Control | 5% Gum arabic solution 1 ml x 5 | + | + | alive |
| | " | + | + | alive |

Note: + indicates detection of fluke egg, and

- no detection.

2) Preparation C

Preparation Example C1 (Tablet and giant pill)

Compound No. C9 (20 g) and 78 g of calcium carbonate were milled and mixed well in a ball mill. 2 g of magnesium stearate was further added thereto, and the mixture was further milled and mixed. The mixture was fed into a tabletting machine to produce tablets each of 1 g (200 mg as effective ingredient). This tablet was administered orally at various doses from 0.2 to 250 g per day depending on the body weight of an animal. By varying the shapes of the mortar and the pestle of the tabletting machine, giant pills can be produced according to the same procedure as described above.

Preparation Example C2 (Liquid preparation)

A liquid preparation was prepared by mixing with stirring 50 g of Compound No.C2, 49 g of ethanol and 1 g of polyvinyl alcohol. This liquid preparation was mixed with stirring into the drinking water for an animal to a concentration of 1% to be suspended in the drinking water, and the resultant suspension was administered to the subject animal.

Preparation Example C3 (Feed mixture)

A solution of 20 g of Compound No.C10 in 20 ml of ethanol was further added to 80 g of lactose and mixed therewith with stirring, and thereafter the ethanol was evaporated off under reduced pressure to be completely

removed. The mixture was mixed into feed for an animal in an amount of 50 g to 2,500 g per ton of the feed depending on the quantity of infecting parasites in the animal. In this case, the proportion of the present compound in feed was 0.001 to 0.05% by weight.

Preparation Example C4 (Capsule)

Compound No. C7 (20 g) and 78 g of calcium carbonate were milled and mixed well in a ball mill. 2 g of magnesium stearate was further added thereto, and the mixture was further milled and mixed. The powder of this mixture was sub-divided, and each sub-division in an amount of 0.5 g was placed into a hard gelatin capsule. In each capsule was contained 100 mg of the present compound. One to 10 capsules per 10 Kg of body weight were administered to an animal depending on its body weight. In this case, the dose of administration of the present compound to the animal was 10 mg to 100 mg per Kg of body weight.

Preparation Example C5 (preparation for injection)

To 20 g of Compound No. C1 were added 1 g of sodium carboxymethyl cellulose, 0.2 g of sodium citrate and distilled water for injection to make up a total quantity of 100 ml, and the mixture was thoroughly mixed with stirring, to prepare a suspension for injection. These preparations for injection were injected subcutaneously, intramuscularly, intraperitoneally or intravaneously in amounts varied from 0.01 ml to 50 ml per day depending on the body weight of the animal.

3) Anthelmintic effect C

Test Example C1

(1) Experimental method

The same as that in Test Example A1 except that the compound provided for testing was in quantities of 30 mg and 50 mg per Kg of body weight, which were orally administered to the chickens in gelatin capsules.

(2) Experimental results

As shown in Table C2, marked anthelmintic effect was observed in every compound of N-alkenyl-N-acyl-O-alkenylhydroxylamine derivatives of the present invention.

The representation of anthelmintic effect was the same as that in Test Example A1.

Table C2

| Compound No. \ Dose (mg/Kg) \ Chicken No. \ Anthelmintic effect | | Anthelmintic effect after 48 hours | | |
|---|---|---|---|---|
| | | No.1 | No.2 | No.3 |
| C1 | 30 | +++ | +++ | +++ |
| | 50 | +++ | +++ | +++ |
| C2 | 30 | +++ | +++ | +++ |
| | 50 | +++ | +++ | +++ |
| C3 | 30 | ++ | ++ | +++ |
| | 50 | +++ | +++ | +++ |
| C4 | 30 | ++ | ++ | +++ |
| | 50 | +++ | +++ | +++ |
| C5 | 30 | ++ | ++ | ++ |
| | 50 | ++ | +++ | +++ |
| C6 | 30 | +++ | +++ | +++ |
| | 50 | +++ | +++ | +++ |
| C7 | 30 | +++ | +++ | +++ |
| | 50 | +++ | +++ | +++ |
| C8 | 30 | +++ | +++ | +++ |
| | 50 | +++ | +++ | +++ |
| C9 | 30 | +++ | +++ | +++ |
| | 50 | +++ | +++ | +++ |
| C10 | 30 | ++ | ++ | ++ |
| | 50 | +++ | +++ | +++ |
| Control | 0 | - | - | - |

Test Example C2

(1) Experimental method

The same as that in Test Example A2, except that the compound provided for the test was added to chicken feed at levels of 25 ppm and 50 ppm.

(2) Experimental results

As shown in Table C3, marked anthelmintic effect was observed in every compound of N-alkenyl-N-acyl-O-alkenyl-hydroxylamine derivatives of the formula (I-3).

The representation of anthelmintic effect was the same as that described above.

Table C3

| Compound No. | Amount added (ppm) | Chicken No. | Change of E.P.G. Before administration | 7th day | 14th day | 21st day | Anthelmintic effect |
|---|---|---|---|---|---|---|---|
| C1 | 25 | 1 | 4,700 | 700 | 100 | 0 | +++ |
| | | 2 | 15,600 | 1,800 | 100 | 0 | +++ |
| | 50 | 1 | 6,500 | 700 | 0 | 0 | +++ |
| | | 2 | 11,400 | 1,300 | 500 | 0 | +++ |
| C2 | 25 | 1 | 31,300 | 10,600 | 2,100 | 0 | +++ |
| | | 2 | 19,400 | 7,400 | 700 | 0 | +++ |
| | 50 | 1 | 11,800 | 2,300 | 500 | 0 | +++ |
| | | 2 | 21,000 | 800 | 0 | 0 | +++ |
| C3 | 25 | 1 | 9,500 | 8,000 | 2,300 | 1,300 | ++ |
| | | 2 | 10,800 | 4,300 | 1,000 | 200 | ++ |
| | 50 | 1 | 26,800 | 2,700 | 500 | 0 | +++ |
| | | 2 | 19,300 | 300 | 0 | 0 | +++ |
| C4 | 25 | 1 | 21,400 | 11,000 | 13,600 | 900 | ++ |
| | | 2 | 9,200 | 5,400 | 1,000 | 100 | ++ |
| | 50 | 1 | 14,600 | 1,200 | 100 | 0 | +++ |
| | | 2 | 13,000 | 300 | 0 | 0 | +++ |
| C5 | 25 | 1 | 5,600 | 3,100 | 1,000 | 100 | ++ |
| | | 2 | 7,900 | 3,000 | 800 | 300 | ++ |
| | 50 | 1 | 15,600 | 1,800 | 500 | 0 | +++ |
| | | 2 | 14,600 | 1,200 | 100 | 0 | +++ |
| C6 | 25 | 1 | 13,000 | 300 | 0 | 0 | +++ |
| | | 2 | 10,700 | 4,300 | 0 | 0 | +++ |
| | 50 | 1 | 21,400 | 11,000 | 1,300 | 0 | +++ |
| | | 2 | 27,600 | 3,300 | 0 | 0 | +++ |

(continued)

| | | | | | | | |
|---|---|---|---|---|---|---|---|
| C7 | 25 | 1 | 17,200 | 300 | 0 | 0 | +++ |
| | | 2 | 8,800 | 3,100 | 100 | 0 | +++ |
| | 50 | 1 | 9,300 | 0 | 0 | 0 | +++ |
| | | 2 | 13,700 | 1,200 | 0 | 0 | +++ |
| C8 | 25 | 1 | 7,600 | 5,800 | 1,000 | 0 | +++ |
| | | 2 | 25,600 | 5,300 | 1,000 | 0 | +++ |
| | 50 | 1 | 4,700 | 700 | 100 | 0 | +++ |
| | | 2 | 15,600 | 2,700 | 100 | 0 | +++ |
| C9 | 25 | 1 | 10,600 | 4,300 | 1,300 | 0 | +++ |
| | | 2 | 9,500 | 4,800 | 900 | 0 | +++ |
| | 50 | 1 | 19,400 | 1,300 | 500 | 0 | +++ |
| | | 2 | 7,400 | 300 | 0 | 0 | +++ |
| C10 | 25 | 1 | 10,600 | 7,600 | 2,000 | 500 | ++ |
| | | 2 | 8,900 | 3,100 | 1,300 | 200 | ++ |
| | 50 | 1 | 21,400 | 9,400 | 2,600 | 0 | +++ |
| | | 2 | 9,300 | 1,200 | 600 | 0 | +++ |
| Con-trol | 0 | 1 | 18,200 | 20,600 | 15,300 | 21,300 | - |
| | | 2 | 7,400 | 13,800 | 15,600 | 13,200 | - |

Test Example C3

(1) Experimental method

The same method as in Test Example A3, but the compound provided for the test was Compound No. Cl. 30 mg/Kg of this compound was placed in a gelatin capsule per 1 Kg of body weight and administered orally to the two dogs. As control, only gelatin capsules were administered orally to the one dog.

(2) Experimental results

As shown in Table C4, marked anthelmintic effect was observed with one administration of 30 mg/Kg of the Compound Cl.

Table C4

Test of anthelmintic effect against canine whipworm, canine hookworm and canine roundworm

| Dose (mg/Kg) | Body weight of dog (Kg) | Kind of parasite | Change of E.P.G. | |
|---|---|---|---|---|
| | | | Before administration | 7th day |
| 30 | 5.5 | Trichuris vulpis | 2,300 | 0 |
| | | Ancylostoma caninum | 4,500 | 0 |
| | | Toxocara canis | 3,300 | 0 |
| 30 | 9.5 | Trichuris vulpis | 4,200 | 0 |
| | | Ancylostoma caninum | 6,600 | 0 |
| | | Toxocara canis | 3,100 | 0 |
| Control | 7.6 | Trichuris vulpis | 2,200 | 1,900 |
| | | Ancylostoma caninum | 7,500 | 4,800 |
| | | Toxocara canis | 1,200 | 1,500 |

Test Example C4

(1) Experimental method

The same as that in Test Example A4, but the compound provided for the test was Compound No. C10, 30 mg/Kg of which was placed in a gelatin capsule per 1 Kg of body weight and administered orally to the two dogs. As control, only gelatin capsules were administered orally to the one dog.

(2) Experimental results

As shown in Table C5, marked anthelmintic effect was observed with one administration of 30 mg/Kg of the compound C10.

Table C5

Test of anthelmintic effect against canine strongyloides

| Dose (mg/Kg) | Body weight of dog (Kg) | Kind of parasite | Change of E.P.G. | |
|---|---|---|---|---|
| | | | Before administration | 7th day |
| 30 | 8.5 | Strongyloides stercoralis | 2,300 | 0 |
| 30 | 10.4 | " | 3,100 | 0 |
| control | 7.3 | " | 900 | 600 |

Test Example C5

(1) Experimental method

The same as in Test Example A5, but the compounds provided for the test were Compound Nos. C2 and C7. 50 mg of each of these compounds per Kg of body weight was placed in a gelatin capsule and administered orally to each of two cats once per day continuously for 5 days. As control, to the two of the cats, was administered orally only one gelatin capsule per day for 5 days.

(2) Experimental results

As shown in Table C7, marked anthelmintic effects were observed with five, continuous administrations of 50 mg/Kg each of the respective compounds of Compound Nos. C2 and C7.

Table C7

Test of anthelmintic effect against Diphyllobothrium erinacei

| Compound No. | Dose mg/Kg x times | Body weight of experimental cat (Kg) | Worm egg detection before administration | Worm egg detection on 10th day after administration | Number of worm bodies remaining in enteron |
|---|---|---|---|---|---|
| C2 | 50 x 5 | 2.3 | + | - | 0 |
| | " | 2.1 | + | - | 0 |
| C7 | " | 2.0 | + | - | 0 |
| | " | 2.0 | + | - | 0 |
| Control | Only gelatin capsule x 5 | 1.8 | + | + | 4 |
| | " | 1.9 | + | + | 2 |

In the Table, + indicates detection of Diphyllobothrium's egg, and - no detection.

Test Example C6

(1) Experimental method

The same as that in Test Example A6, but the compound provided for the test was Compound No. C1, 20 mg of which was placed in a gelatin capsule per 1 Kg of body weight and administered orally to the two cows. As control, only gelatin capsules were administered orally to the one cow.

(2) Experimental results

As shown in Table C8, marked anthelmintic effect was observed with one administration of 20 mg/Kg of the Compound C1.

Table C8

Test of anthelmintic effect against cow stomach worm

| Dose (mg/Kg) | Body weight of cow (Kg) | Kind of parasite | Change of E.P.G. | |
|---|---|---|---|---|
| | | | Before administration | 7th day |
| 20 | 420 | Haemonchus controtus | 2,200 | 0 |
| 20 | 380 | " | 1,400 | 0 |
| control | 450 | " | 800 | 1,300 |

Test Example C7

(1) Experimental method

The same as that in Test Example A7, but the compound provided for the test was Compound No.C10, 20 mg of which was placed in a gelatin capsule per 1 Kg of body weight and administered orally to one of the sheep. As control, only gelatin capsule was administered orally to the other sheep.

(2) Experimental results

As shown in Table C9, marked anthelmintic effect was observed with one administration of 15 mg/Kg of the compound C10.

Table C9

Test of anthelmintic effect against sheep Trichostrongyldae

| Dose (mg/Kg) | Body weight of sheet (Kg) | Kind of parasite | Change of E.P.G. | |
|---|---|---|---|---|
| | | | Before administration | 7th day |
| 20 | 42 | Trichostrongylus colubriformis | 1,800 | 0 |
| control | 56 | " | 900 | 800 |

Test Example C8

(1) Experimental method

The same as that in Test Example A8, except that the compounds provided for the test were Compound Nos. C2 and C6.

(2) Experimental results

As shown in Table C10, marked anthelmintic effect was observed with one administration of 20 mg/Kg of the compounds C2 and C6, respectively.

Table C10

Test of anthelmintic effect against swine Oesophagostomum

| Compound No. | Dose (mg/Kg) | Body weight of pig (Kg) | Kind of parasite | Change of E.P.G. Before administration | Change of E.P.G. 7th day |
|---|---|---|---|---|---|
| C2 | 20 | 65 | Oesophagostomum dentatum | 1,700 | 0 |
| | 20 | 70 | " | 1,200 | 0 |
| C6 | 20 | 58 | " | 3,300 | 0 |
| | 20 | 60 | " | 1,800 | 0 |
| Control | Only gelatin capsule | 75 | " | 1,200 | 1,500 |
| | | 70 | " | 900 | 1,000 |

Test Example C9

(1) Experimental method

The same as that in Test Example A9, but the compounds provided for the test were Compound Nos. C3 and C6. 30 mg of each compound was placed in a gelatin capsule per 1 Kg of body weight and administered orally to each of the two pigs. As control, only gelatin capsules were administered orally to two pigs.

(2) Experimental results

As shown in Table C11, marked anthelmintic effect was observed with one administration of 30 mg/Kg of the Compounds C3 and C6, respectively.

Table C11

Test of anthelmintic effect
against swine roundworm and swine whipworm

| Compound No. | Dose (mg/Kg) | Body weight of pig (Kg) | Kind of parasite | Change of E.P.G. | |
|---|---|---|---|---|---|
| | | | | Before administration | 7th day |
| C3 | 30 | 71 | Ascaris suum | 1,300 | 0 |
| | | | Trichuris suis | 6,600 | 0 |
| | 30 | 68 | Ascaris suum | 2,100 | 0 |
| | | | Trichuris suis | 1,900 | 0 |
| C6 | 30 | 63 | Ascaris suum | 8,300 | 0 |
| | | | Trichuris suis | 5,800 | 0 |
| | 30 | 65 | Ascaris suum | 700 | 0 |
| | | | Trichuris suis | 4,300 | 0 |
| Control | Only gelatin capsule | 55 | Ascaris suum | 1,200 | 800 |
| | | | Trichuris suis | 2,100 | 2,000 |
| | Only gelatin capsule | 50 | Ascaris suum | 300 | 200 |
| | | | Trichuris suis | 500 | 700 |

Test Example C10

(1) Experimental method

The same as that in Test Example A10, but the compounds provided for tests were Compound Nos. C2, C1 and C9. 30 mg of each compound was administered by subcutaneous injection per Kg of body weight to each of two dogs as a suspension in 1% "Tween 80" solution once per day for three days. As control, 3 ml of the 1% "Tween 80" solution was subcutaneously injected to two dogs once per day for three days.

(2) Experimental results

As shown in Table C12, marked anthelmintic effect was observed against microfilaria (mf) in each of the compounds of Compound Nos.C2, C1 and C9 by administration of 30 mg/Kg each repeated three times, while about 50% or more of anthelmintic effect was observed against adult filaria.

Table C12

Test of anthelmintic effect against canine filaria

| Compound No. | Dose x Times mg/Kg x Times | Body weight of dog (Kg) | Number of microfilaria/20 μl | | Effect on adult filaria | |
|---|---|---|---|---|---|---|
| | | | Before administration | On 10th day after the last administration | Number of worms killed/ total parasite | Percent of worms killed |
| C2 | 30 x 3 | 12.5 | 35 | 0 | 5/8 | 62 |
| | 30 x 3 | 15.3 | 18 | 0 | 8/13 | 61 |
| C1 | 30 x 3 | 10.8 | 23 | 0 | 13/15 | 86 |
| | 30 x 3 | 11.6 | 31 | 0 | 16/21 | 76 |
| C9 | 30 x 3 | 13.3 | 40 | 0 | 14/20 | 70 |
| | 30 x 3 | 12.5 | 17 | 0 | 19/27 | 70 |
| Control | 1% [Tween 80] | 7.1 | 15 | 11 | 0/9 | 0 |
| | 3ml x 3 | 8.4 | 18 | 21 | 0/13 | 0 |

Test Example C11

(1) Experimental method

The same as that in Test Example A11, but six (6) rats were employed by grouping them into groups each of two rats, each rat being infected with 5 metacercariae of Fasciola hepatica and confirmed to have worm eggs on the 75th day after infection according to fecal matter testing.

The compounds provided for the tests were Compound Nos. C2 and C6.

(2) Experimental results

As shown in Table C13, marked antihelmintic effect was observed in each compound of Compound Nos. C2 and C6 with five administrations in a dose of 50 mg/Kg each.

Table C13

Test of anthelmintic effect against rat infected experimentally with liver fluke (F. hepatica)

| Compound No. | Dose x Times mg/Kg x Times | Presence or absence of fluke egg in fecal matter | | Condition of fluke body |
|---|---|---|---|---|
| | | Before administration | On 21st day after the last administration | |
| C2 | 50 x 5 | + | - | dead |
| | 50 x 5 | + | - | dead |
| C6 | 50 x 5 | + | - | dead |
| | 50 x 5 | + | - | dead |
| Control | 5% Gum arabic solution 1 ml x 5 | + | + | alive |
| | 5% Gum arabic solution 1 ml x 5 | + | + | alive |

Note: + indicates detection of fluke egg, and - no detection.

2) Preparation D

Preparation Example D1 (Tablet and giant pill)

Compound No.D1 (20 g) and 78 g of calcium carbonate were milled and mixed well in a ball mill. 2 g of magnesium stearate was added further thereto, and the mixture was further milled and mixed. The mixture was fed into a tabletting machine to produce tablets each of 1g (200 mg as effective ingredient). This tablet was administered orally in various doses from 0.2 to 250 g per day depending on the body weight of the animal. By varying the shapes of the mortar and the pestle of the tabletting machine, giant pills can be produced according to the same procedure as described above.

Preparation Example D2 (Liquid preparation)

A liquid preparation was prepared by adding 10 g of distilled water to 10 g of Compound No. D1, which step was followed by mixing with stirring. This liquid preparation was added into the drinking water for the animal to a concentration of 5%, which step was followed by mixing with stirring, to be dissolved in the drinking water, and the resultant solution was administered to the subject animal.

Preparation Example D3 (Feed mixture)

Twenty grams (20 g) of Compound No. D10 were added to 80 g of lactose and mixed therewith with stirring, and the mixture was mixed into feed for the animal in an amount of 50 g to 2,500 g per ton of the feed depending

on the quantity of infecting parasites in the animal. In this case, the proportion of the present compound in the feed is 0.001 to 0.05% by weight.

Preparation Example D4 (Capsule)

Compound No. D13 (20 g) and 78 g of calcium carbonate were milled and mixed well in a ball mill. 2 g of magnesium stearate was further added thereto, and the mixture was further milled and mixed. The powder of this mixture was sub-divided, and each sub-division in an amount of 0.5 g was placed in a hard gelatin capsule. In each capsule was contained 100 mg of the present compound. One to 10 capsules per 10 Kg of body weight were administered to the animal depending on its body weight. In this case, the dose of administration of the present compound to the animal is 10 mg to 100 mg per Kg of body weight.

Preparation Example D5 (preparation for injection)

To 20 g of Compound No. D9 were added 1 g of sodium carboxymethyl cellulose, 0.2 g of sodium citrate and distilled water for injection to make up a total quantity of 100 ml, and the mixture was thoroughly mixed with stirring to prepare a suspension for injection. These preparations for injection were injected subcutaneously, intramuscularly, intraperitoneally or intravaneously in amounts varied from 0.01 ml to 50 ml per day depending on the body weight of the animal.

0103895

3) Anthelmintic effect D

Test Example D1

(1) Experimental method

The same as that in Test Example A1, except that the compounds provided for testing were used in quantities of 15 mg and 30 mg per Kg of body weight, which were orally administered each in a gelatin capsule to the chicken.

As shown in Table D2, marked anthelmintic effect was observed in every compound of hydroxylamine derivatives of the present invention.

The representation of anthelmintic effect was the same as that in Test Example A1.

Table D2

| Compound No. | Anthelmintic effect / Chicken No. / Dose (mg/Kg) | Anthelmintic effect after 48 hours | | |
|---|---|---|---|---|
| | | No.1 | No.2 | No.3 |
| D1 | 15 | ++ | ++ | +++ |
| | 30 | +++ | +++ | +++ |
| D2 | 15 | ++ | + | ++ |
| | 30 | +++ | +++ | +++ |
| D3 | 15 | + | + | ++ |
| | 30 | +++ | +++ | +++ |
| D4 | 15 | ++ | +++ | ++ |
| | 30 | +++ | +++ | +++ |
| D5 | 15 | +++ | +++ | +++ |
| | 30 | +++ | +++ | +++ |
| D6 | 15 | ++ | +++ | +++ |
| | 30 | +++ | +++ | +++ |
| D7 | 15 | ++ | + | + |
| | 30 | +++ | +++ | +++ |
| D8 | 15 | ++ | +++ | ++ |
| | 30 | +++ | +++ | +++ |
| D9 | 15 | + | + | + |
| | 30 | +++ | +++ | +++ |
| D10 | 15 | +++ | +++ | +++ |
| | 30 | +++ | +++ | +++ |
| D11 | 15 | + | ++ | ++ |
| | 30 | +++ | +++ | +++ |
| D12 | 15 | ++ | + | ++ |
| | 30 | +++ | +++ | +++ |
| D13 | 15 | ++ | ++ | ++ |
| | 30 | +++ | +++ | +++ |

(continued)

| | | | | |
|---|---|---|---|---|
| D14 | 15<br>30 | ++<br>+++ | ++<br>+++ | ++<br>+++ |
| D15 | 15<br>30 | +++<br>+++ | +++<br>+++ | +++<br>+++ |
| D16 | 15<br>30 | +++<br>+++ | +++<br>+++ | +++<br>+++ |
| Control | 0 | - | - | - |

Test Example D2

(1) Experimental method

The same as that in Test Example A2, but in the case of a liquid compound provided for the test, it was adsorbed on lactose as a solution dissolved in a small amount of ethyl alcohol, while in the case of a solid compound, it was mixed with lactose, and added to chicken feed at levels of 25 ppm and 50 ppm.

(2) Experimental results

As shown in Table D3, marked anthelmintic effect was observed in every compound of N-propargyl-N-acylhydroxylamine derivatives of the formula (I-4).

The representation of anthelmintic effect was the same as that described above.

Table D3

| Compound No. | Amount added (ppm) | Chicken No. | Change of E.P.G. Before administration | 7th day | 14th day | 21st day | Anthelmintic effect |
|---|---|---|---|---|---|---|---|
| D1 | 25 | 1 | 6,600 | 3,100 | 800 | 300 | ++ |
| | | 2 | 13,200 | 1,800 | 100 | 0 | +++ |
| | 50 | 1 | 7,500 | 900 | 0 | 0 | +++ |
| | | 2 | 13,300 | 2,100 | 300 | 0 | +++ |
| D2 | 25 | 1 | 23,100 | 8,400 | 1,200 | 600 | ++ |
| | | 2 | 16,500 | 4,700 | 800 | 200 | ++ |
| | 50 | 1 | 12,300 | 3,200 | 300 | 0 | +++ |
| | | 2 | 21,400 | 900 | 0 | 0 | +++ |
| D3 | 25 | 1 | 8,300 | 8,400 | 2,200 | 1,000 | ++ |
| | | 2 | 12,400 | 5,400 | 1,000 | 400 | ++ |
| | 50 | 1 | 21,800 | 4,200 | 900 | 0 | +++ |
| | | 2 | 14,600 | 1,200 | 200 | 0 | +++ |
| D4 | 25 | 1 | 22,500 | 11,200 | 5,300 | 800 | ++ |
| | | 2 | 9,300 | 4,500 | 800 | 200 | ++ |
| | 50 | 1 | 16,400 | 2,300 | 100 | 0 | +++ |
| | | 2 | 12,900 | 1,300 | 300 | 0 | +++ |
| D5 | 25 | 1 | 6,200 | 2,800 | 600 | 600 | ++ |
| | | 2 | 9,700 | 3,500 | 1,100 | 200 | ++ |
| | 50 | 1 | 16,600 | 2,100 | 400 | 0 | +++ |
| | | 2 | 16,400 | 1,900 | 2,100 | 0 | +++ |
| D6 | 25 | 1 | 12,300 | 4,200 | 1,000 | 100 | ++ |
| | | 2 | 7,400 | 2,100 | 600 | 0 | +++ |
| | 50 | 1 | 22,600 | 9,200 | 1,200 | 0 | +++ |
| | | 2 | 27,400 | 3,200 | 0 | 0 | +++ |

(continued)

| | | | | | | | |
|---|---|---|---|---|---|---|---|
| D7 | 25 | 1 | 8,300 | 2,200 | 600 | 600 | ++ |
| | | 2 | 12,400 | 7,700 | 2,100 | 1,000 | ++ |
| | 50 | 1 | 9,900 | 0 | 0 | 0 | +++ |
| | | 2 | 29,200 | 1,600 | 0 | 0 | +++ |
| D8 | 25 | 1 | 4,400 | 5,100 | 1,000 | 100 | ++ |
| | | 2 | 10,900 | 4,300 | 2,600 | 700 | ++ |
| | 50 | 1 | 9,600 | 800 | 0 | 0 | +++ |
| | | 2 | 17,300 | 1,600 | 1,200 | 0 | +++ |
| D9 | 25 | 1 | 11,300 | 4,300 | 1,300 | 0 | +++ |
| | | 2 | 9,600 | 5,700 | 3,600 | 1,200 | ++ |
| | 50 | 1 | 11,800 | 1,300 | 500 | 0 | +++ |
| | | 2 | 7,900 | 800 | 0 | 0 | +++ |
| D10 | 25 | 1 | 10,400 | 8,600 | 2,100 | 600 | ++ |
| | | 2 | 9,800 | 1,300 | 1,300 | 700 | ++ |
| | 50 | 1 | 26,400 | 8,300 | 2,200 | 0 | +++ |
| | | 2 | 12,700 | 1,200 | 0 | 0 | +++ |
| D11 | 25 | 1 | 15,500 | 8,600 | 1,300 | 2,100 | ++ |
| | | 2 | 12,500 | 7,700 | 5,300 | 600 | ++ |
| | 50 | 1 | 21,800 | 2,600 | 300 | 0 | +++ |
| | | 2 | 22,900 | 900 | 0 | 0 | +++ |
| D12 | 25 | 1 | 15,300 | 4,700 | 2,100 | 300 | ++ |
| | | 2 | 8,900 | 3,300 | 1,800 | 700 | ++ |
| | 50 | 1 | 26,600 | 2,400 | 300 | 0 | +++ |
| | | 2 | 16,400 | 900 | 0 | 0 | +++ |
| D13 | 25 | 1 | 18,800 | 11,200 | 6,300 | 900 | ++ |
| | | 2 | 8,300 | 4,200 | 800 | 100 | ++ |
| | 50 | 1 | 32,600 | 2,800 | 0 | 0 | +++ |
| | | 2 | 18,300 | 3,100 | 100 | 0 | +++ |

(continued)

0103895

| | | | | | | | |
|---|---|---|---|---|---|---|---|
| D14 | 25 | 1 | 12,700 | 11,600 | 5,400 | 1,200 | ++ |
| | | 2 | 7,300 | 1,600 | 800 | 800 | ++ |
| | 50 | 1 | 13,600 | 800 | 100 | 0 | +++ |
| | | 2 | 9,800 | 2,200 | 300 | 0 | +++ |
| D15 | 25 | 1 | 6,700 | 1,200 | 800 | 700 | ++ |
| | | 2 | 9,400 | 3,100 | 100 | 0 | +++ |
| | 50 | 1 | 12,800 | 100 | 0 | 0 | +++ |
| | | 2 | 17,900 | 2,100 | 100 | 0 | +++ |
| D16 | 25 | 1 | 16,500 | 1,300 | 900 | 0 | +++ |
| | | 2 | 8,700 | 2,100 | 100 | 100 | ++ |
| | 50 | 1 | 13,500 | 1,100 | 0 | 0 | +++ |
| | | 2 | 15,400 | 500 | 0 | 0 | +++ |
| Control | 0 | 1 | 13,400 | 20,100 | 19,300 | 16,200 | - |
| | | 2 | 9,800 | 13,400 | 17,200 | 11,600 | - |

Test Example D3

(1) Experimental method

The same as that in Test Example A3, except that the compound provided for the test was Compound No.D14.

(2) Experimental results

As shown in Table D4, marked anthelmintic effect was observed with one administration of 20 mg/Kg of the Compound D14.

Table D4

Test of anthelmintic effect against canine whipworm, canine hookworm and canine roundworm .

| Dose (mg/Kg) | Body weight of dog (Kg) | Kind of parasite | Change of E.P.G. Before administ-ration | 7th day |
|---|---|---|---|---|
| 20 | 8.9 | Trichuris vulpis | 4,600 | 0 |
| | | Ancylostoma caninum | 3,300 | 0 |
| | | Toxocara canis | 7,200 | 0 |
| 20 | 11.6 | Trichuris vulpis | 1,300 | 0 |
| | | Ancylostoma caninum | 6,800 | 0 |
| | | Toxocara canis | 2,200 | 0 |
| Control | 5.4 | Trichuris vulpis | 1,300 | 2,100 |
| | | Ancylostoma caninum | 2,100 | 2,200 |
| | | Toxocara canis | 1,900 | 1,200 |

Test Example D4

(1) Experimental method

The same as that in Test Example A4, except that the compound provided for the test Compound No. D16.

(2) Experimental results

As shown in Table D5, marked anthelmintic effect was observed with one administration of 10 mg/Kg of the Compound D16.

Table D5

Test of anthelmintic effect against canine strongyloides

| Dose (mg/Kg) | Body weight of dog (Kg) | Kind of parasite | Change of E.P.G. | |
|---|---|---|---|---|
| | | | Before administration | 7th day |
| 10 | 7.5 | Strongyloides stercoralis | 2,300 | 0 |
| 10 | 6.4 | " | 1,800 | 0 |
| Control | 8.5 | " | 100 | 300 |

Test Example D5

(1) Experimental method

The same as that in Test Example A5, but the compounds provided for the test were Compound Nos.D9 and D16.

(2) Experimental results

As shown in Table D7, marked anthelmintic effects were observed with five continuous administrations of 50 mg/Kg of the respective Compound Nos. D9 and D16.

Table D7

Test of anthelmintic effect against Diphyllobothrium erinacei

| Compound No. | Dose mg/Kg x Times | Body weight of experimental cat (Kg) | Worm egg detection before administration | Worm egg detection on 10th day after administration | Number of worms remaining in enteron |
|---|---|---|---|---|---|
| D9 | 50 x 5 | 1.8 | + | - | 0 |
| | " | 2.3 | + | - | 0 |
| D16 | " | 2.2 | + | - | 0 |
| | " | 1.6 | + | - | 0 |
| Control | Only gelatin capsule | 1.2 | + | + | 5 |
| | " | 1.7 | + | + | 3 |

In the Table, + indicates detection of Diphyllobothrium's egg, and - no detection.

Test Example D6

(1) Experimental method

The same as that in Test Example A6, but the compound provided for the test was Compound No. D1.

(2) Experimental results

As shown in Table D8, marked anthelmintic effect was observed with one administration of 15 mg/Kg of the Compound No. D1.

Table D8

Test of anthelmintic effect against cow stomach worm

| Dose (mg/Kg) | Body weight of cow (Kg) | Kind of parasite | Change of E.P.G. | |
|---|---|---|---|---|
| | | | Before administration | 7th day |
| 15 | 380 | Haemonchus controtus | 3,700 | 0 |
| 15 | 410 | " | 5,400 | 0 |
| Control | 550 | " | 2,100 | 3,300 |

Test Example D7

(1) Experimental method

The same as that in Test Example A7, but the compound provided for the test was Compound No.D1.

(2) Experimental results

As shown in Table D9, marked anthelmintic effect was observed with one administration of 15 mg/Kg of the Compound D1.

Table D9

Test of anthelmintic effect against sheep Trichostrongyldae

| Dose (mg/Kg) | Body weight of sheep (Kg) | Kind of parasite | Change of E.P.G. | |
|---|---|---|---|---|
| | | | Before administration | 7th day |
| 15 | 56 | Trichostrongylus colubriformis | 2,900 | 0 |
| control | 48 | " | 1,200 | 1,800 |

Test Example D8

(1) Experimental method

The same as that in Test Example A8, but the compounds provided for the test were Compound Nos. D3 and D12.

(2) Experimental results

As shown in Table D10, marked anthelmintic effect was observed with one administration of 20 mg/Kg of the compounds D3 and D12, respectively.

Table D10

Test of anthelmintic effect against swine Oesophagostomum

| Compound No. | Dose (mg/Kg) | Body weight of pig (Kg) | Kind of parasite | Change of E.P.G. Before administration | Change of E.P.G. 7th day |
|---|---|---|---|---|---|
| D3 | 20 | 55 | Oesophagostomum dentatum | 2,200 | 0 |
| | 20 | 48 | " | 1,800 | 0 |
| D12 | 20 | 51 | " | 1,600 | 0 |
| | 20 | 58 | " | 2,100 | 0 |
| Control | Only gelatin capsule | 47 | " | 1,300 | 1,500 |
| | | 50 | " | 1,200 | 1,000 |

Test Example D9

(1) Experimental method

The same as that in Test Example A9, but the compounds provided for the test were Compound Nos. D1 and D10.

(2) Experimental results

As shown in Table D11, marked anthelmintic effect was observed with one administration of 20 mg/Kg of the Compounds A1 and A10, respectively.

Table D11

Test of anthelmintic effect
against swine roundworm and swine whipworm

| Compound No. | Dose (mg/Kg) | Body weight of pig (Kg) | Kind of parasite | Change of E.P.G. Before administration | 7th day |
|---|---|---|---|---|---|
| D1 | 20 | 55 | Ascaris suum | 2,700 | 0 |
| | | | Trichuris suis | 6,600 | 0 |
| | 20 | 50 | Ascaris suum | 3,100 | 0 |
| | | | Trichuris suis | 4,200 | 0 |
| D10 | 20 | 48 | Ascaris suum | 3,800 | 0 |
| | | | Trichuris suis | 5,300 | 0 |
| | 20 | 57 | Ascaris suum | 1,600 | 0 |
| | | | Trichuris suis | 2,200 | 0 |
| Control | Only gelatin capsule | 49 | Ascaris suum | 4,800 | 1,3000 |
| | | | Trichuris suis | 1,600 | 2,100 |
| | Only gelatin capsule | 50 | Ascaris suum | 1,300 | 2,700 |
| | | | Trichuris suis | 1,500 | 1,000 |

Test Example D10

(1) Experimental method

The same as that in Test Example A10, but the compounds provided for tests were Compound Nos. D10, D13 and D16. 20 mg of each compound was administered by subcutaneous injection per kg of body weight once per day for three days to each of two dogs. These compounds were administered as a suspension in 1% "Tween 80" in the case of Compound No.D13 and as solutions dissolved in distilled water for injection in the case of Compounds Nos. D10 and D16, respectively. As control, 3 ml of the 1% "Tween 80" solution was subcutaneously injected into two dogs once per day for three days.

(2) Experimental results

As shown in Table D12, marked anthelmintic effect was observed against microfilaria (mf) in each of the compounds of Compound Nos. D10, D13 and D16 with administration of 20 mg/Kg repeated three times, while about 50% or more of anthelmintic effect was observed against adult filaria.

Table D12

Test of anthelmintic effect against canine filaria

| Compound No. | Dose x Times mg/Kg x Times | Body weight of dog (Kg) | Number of microfilaria/20μl | | Effect on adult filaria | |
|---|---|---|---|---|---|---|
| | | | Before administration | On 10th day after the administration | Number of worms killed/ Total parasite | Percent of worms killed |
| D10 | 20 x 3 | 8.7 | 41 | 0 | 18/33 | 54 |
| | 20 x 3 | 6.4 | 18 | 0 | 20/28 | 71 |
| D13 | 20 x 3 | 10.5 | 33 | 0 | 21/33 | 63 |
| | 20 x 3 | 12.1 | 26 | 0 | 20/30 | 66 |
| D16 | 20 x 3 | 7.3 | 18 | 0 | 16/29 | 55 |
| | 20 x 3 | 5.8 | 31 | 0 | 23/37 | 62 |
| Control | 1% [Tween 80] | 6.5 | 13 | 26 | 0/13 | 0 |
| | 3 ml x 3 | 7.3 | 18 | 13 | 0/41 | 0 |

Test Example D11

(1) Experimental method

The same as that in Test Example A11, but the compounds provided for tests were Compound Nos. D14, D15 and D16.

(2) Experimental results

As shown in Table D13, marked anthelmintic effect was observed in each of the Compound Nos. D14, D15 and D16 with five administrations each in a dose of 50 mg/Kg.

Table D13

Test of anthelmintic effect against rat infected experimentally with liver fluke (F. hepatica)

| Compound No. | Dose x Times mg/Kg x Times | Presence or absence of fluke egg in fecal matter | | Condition of fluke bodies |
|---|---|---|---|---|
| | | Before administration | On 21st day after the last administration | |
| D14 | 50 x 5 | + | - | dead |
| | " | + | - | dead |
| D15 | " | + | - | dead |
| | " | + | - | dead |
| D16 | " | + | - | dead |
| | " | + | - | dead |
| Control | 5% Gum arabic solution 1 ml x 5 | + | + | alive |
| | | + | + | alive |

Note: + indicates detection of fluke egg, and - no detection.

2) Preparation E

Preparation Example E1 (Tablet and giant pill)

The compound of Compound No. E1 (20g) and 78 g of calcium carbonate were milled and mixed well in a ball mill. 2 g of magnesium stearate was further added thereto, and the mixture was further milled and mixed. The mixture was fed into a tabletting machine to produce tablets each of 1 g (200 mg as effective ingredient). This tablet was administered orally in various doses from 0.2 to 250 g per day depending on the body weight of the animal.

By varying the shapes of the mortar and the pestle of the tabletting machine, giant pills can be produced according to the same procedure as described above.

Preparation Example E2 (Liquid preparation)

A liquid preparation was prepared by mixing under stirring 50 g of Compound No. E3, 49 g of ethanol and 1 g of polyvinyl alcohol. This liquid preparation was mixed with stirring into the drinking water for the animal to a concentration of 1% to be suspended in the drinking water, and the resultant suspension was administered to the subject animal.

Preparation Example E3 (Feed mixture)

A solution of 20 g of Compound No. E7 in 20 ml of ethanol was further added to 80 g of defatted rice bran and mixed therewith with stirring, and thereafter ethanol was evaporated off under reduced pressure to be completely

removed. The mixture was mixed into feed for the animal in an amount of 50 g to 1,250 g per ton of the feed depending on the quantity of infecting parasites in the animal. In this case, the proportion of the present compound in the feed was 0.001 to 0.025% by weight.

Preparation Example E4 (Capsule)

Compound No. E5 (20 g) and 78 g of calcium carbonate were milled and mixed well in a ball mill. 2 g of magnesium stearate was further added thereto, and the mixture was further milled and mixed. The powder of this mixture was sub-divided, and each sub-division in an amount of 0.5 g was placed in a hard gelatin capsule. In each capsule was contained 100 mg of the present compound. One to 10 capsules per 10 Kg of body weight were administered to each animal depending on the body weight of the animal. In this case, the dose of administration of the present compound to the animal was 10 mg to 100 mg per Kg of body weight.

Preparation Example E5 (preparation for injection)

To 20 g of Compound No. E6 were added 1 g of sodium carboxymethyl cellulose, 0.2 g of sodium citrate and distilled water for injection to make up a total quantity of 100 ml, and the mixture was thoroughly mixed with stirring, to prepare a suspension for injection. These preparations for injection were injected subcutaneously, intramuscularly, intraperitoneally or intravaneously in amounts varied from 0.01 ml to 50 ml per day depending on the body weight of the animal.

3) <u>Anthelmintic effect E</u>

<u>Test Example E1</u>

(1) Experimental method

Chickens infected with fowl roundworm (As. galli) were employed in groups each consisting of five chickens.

The compound provided for testing was orally administered in a quantity of 40 mg per Kg of body weight to each chicken in a gelatin capsule. The number of worm bodies discharged within 24 hours after administration of the medicine was counted. Then the chicken was killed, and the number of worm bodies remaining in the enteron was counted to determine the anthelmintic percentage, wherefrom the anthelmintic effect was judged.

(2) Experimental results

As shown in Table E2, marked anthelmintic effect was observed in every compound of N,O-dipropargyl-N-acylhydroxylamine derivatives of the present invention.

The representation of anthelmintic effect was the same as in Test Example A1.

0103895

Table E2

| Compound No. \ Chicken No. | Anthelmintic effect after one day | | | | |
|---|---|---|---|---|---|
| | No.1 | No.2 | No.3 | No.4 | No.5 |
| E1 | +++ | +++ | +++ | +++ | +++ |
| E2 | +++ | +++ | +++ | +++ | +++ |
| E3 | +++ | +++ | +++ | +++ | +++ |
| E4 | +++ | +++ | +++ | +++ | +++ |
| E5 | +++ | +++ | +++ | +++ | +++ |
| E6 | +++ | +++ | +++ | +++ | +++ |
| E7 | +++ | +++ | +++ | +++ | +++ |
| E8 | +++ | +++ | +++ | +++ | +++ |
| E9 | +++ | +++ | +++ | +++ | +++ |
| E10 | +++ | +++ | +++ | +++ | +++ |
| E11 | +++ | +++ | +++ | +++ | +++ |
| Control (only gelatin capsule) | - | - | - | - | - |

Test Example E2

(1) Experimental method

Chickens infected with fowl roundworm (As. galli) were employed in groups each of two chickens. The compound provided for the test was dissolved in a small amount of ethyl alcohol and adsorbed on defatted rice bran and added to chicken feed at levels of 250 ppm and 1,000 ppm. As control, a feed containing no such compound was employed.

Fecal matter testing was conducted before initiation of the test and on the 5th day after initiation of the test. The reduction in worm egg count was examined by calculating the E.P.G. (worm egg number in 1 g of fecal matter). The number of discharged worm bodies and the number of worm bodies remaining in the enteron when the chicken was killed on 5th day were counted to determined the anthelmintic percentage, from which the anthelmintic effect was judged.

(2) Experimental result

As shown in Table E3, marked anthelmintic effect was observed in every compound of N,O-dipropargyl-N-acylhydroxylamine derivatives.

Table E3

| Compound No. | Amount added | Chicken No. | E.P.G. before administration | E.P.G. after 5 days | Anthelmintic effect |
|---|---|---|---|---|---|
| E1 | 1000 ppm | 1 | 26,500 | 0 | +++ |
| | | 2 | 23,000 | 0 | +++ |
| | 250 ppm | 1 | 24,300 | 0 | +++ |
| | | 2 | 22,200 | 0 | +++ |
| E2 | 1000 ppm | 1 | 18,900 | 0 | +++ |
| | | 2 | 25,400 | 0 | +++ |
| | 250 ppm | 1 | 23,700 | 0 | +++ |
| | | 2 | 19,200 | 0 | +++ |
| E3 | 1000 ppm | 1 | 35,600 | 0 | +++ |
| | | 2 | 21,300 | 0 | +++ |
| | 250 ppm | 1 | 20,700 | 0 | +++ |
| | | 2 | 18,300 | 0 | +++ |
| E4 | 1000 ppm | 1 | 43,800 | 0 | +++ |
| | | 2 | 21,600 | 0 | +++ |
| | 250 ppm | 1 | 37,800 | 0 | +++ |
| | | 2 | 31,600 | 0 | +++ |
| E5 | 1000 ppm | 1 | 19,800 | 0 | +++ |
| | | 2 | 28,400 | 0 | +++ |
| | 250 ppm | 1 | 33,300 | 0 | +++ |
| | | 2 | 26,400 | 0 | +++ |
| E6 | 1000 ppm | 1 | 22,800 | 0 | +++ |
| | | 2 | 21,300 | 0 | +++ |
| | 250 ppm | 1 | 26,700 | 0 | +++ |
| | | 2 | 27,100 | 0 | +++ |

(continued)

| | | | | | |
|---|---|---|---|---|---|
| E7 | 1000 ppm | 1 | 43,600 | 0 | +++ |
| | | 2 | 30,700 | 0 | +++ |
| | 250 ppm | 1 | 31,800 | 0 | +++ |
| | | 2 | 28,400 | 0 | +++ |
| E8 | 1000 ppm | 1 | 22,100 | 0 | +++ |
| | | 2 | 19,600 | 0 | +++ |
| | 250 ppm | 1 | 17,400 | 0 | +++ |
| | | 2 | 25,600 | 0 | +++ |
| E9 | 1000 ppm | 1 | 31,300 | 0 | +++ |
| | | 2 | 29,000 | 0 | +++ |
| | 250 ppm | 1 | 34,300 | 0 | +++ |
| | | 2 | 43,200 | 0 | +++ |
| E10 | 1000 ppm | 1 | 25,600 | 0 | +++ |
| | | 2 | 22,000 | 0 | +++ |
| | 250 ppm | 1 | 20,300 | 0 | +++ |
| | | 2 | 20,100 | 0 | +++ |
| E11 | 1000 ppm | 1 | 33,500 | 0 | +++ |
| | | 2 | 26,200 | 0 | +++ |
| | 250 ppm | 1 | 23,700 | 0 | +++ |
| | | 2 | 18,300 | 0 | +++ |
| Con-trol | No addition | 1 | 32,800 | 27,800 | - |
| | | 2 | 34,300 | 45,800 | - |

WHAT IS CLAIMED IS:

1. A hydroxylamine derivative represented by the formula

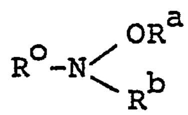 (A)

where: $R^o$ is a hydrogen atom or a group of the formula $R^1$-CO- where $R^1$ is a hydrogen atom or an alkyl or alkenyl having up to 5 carbon atoms, phenyl, or a substituted phenyl, the substituent being an alkyl having up to 3 carbon atoms, a halogen atom or nitro,

$R^a$ is an alkyl, alkenyl or alkynyl having up to 5 carbon atoms; and

$R^b$ is an alkenyl or alkynyl having up to 5 carbon atoms,

the combination of $R^o$, $R^a$ and $R^b$ being selected from the group consisting of:

(1) the combination in which

$R^o$ is $R^1$-CO-, $R^1$ being defined hereinabove,

$R^a$ is an alkyl or alkenyl having up to 5 carbon atoms, and

$R^b$ is $-CH_2C{\equiv}CH$,

provided that $R^a$ is not an alkyl when $R^1$ is phenyl;

(2) the combination in which

$R^o$ is $R^1$-CO-, $R^1$ being as defined hereinabove,

$R^a$ is an alkyl having up to 5 carbon atoms, and

$R^b$ is $-CH_2CH=CH-R^3$, $R^3$ being hydrogen or methyl,

provided that $R^a$ is not an alkyl having 3 carbon atoms when $R^1$ is phenyl;

(3) -

(4) the combination in which

$R^o$ is H,

$R^a$ is as defined hereinabove, and

$R^b$ is as defined hereinabove,

provided that $R^a$ is not methyl, or $R^a$ and $R^b$ are not allyl simultaneously; and

(5) the combination in which

$R^o$ is $R^1$-CO-, $R^1$ being as defined hereinabove,

$R^a$ is $-CH_2C{\equiv}CH$ and

$R^b$ is $-CH_2C{\equiv}CH$.

2. The compound as claimed in claim 1 wherein the substituent in $R^1$ is an alkyl having up to 3 carbon atoms or a halogen atom when $R^1$ is a substituted phenyl.

3. The compound as claimed in claim 1 wherein $R^1$ is an alkyl having up to 5 carbon atoms.

4. The cmopund as claimed in claim 3 selected from the group consisting of:

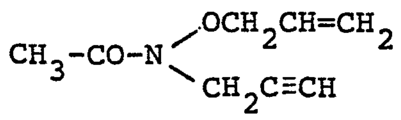,

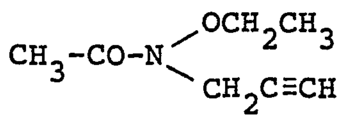,

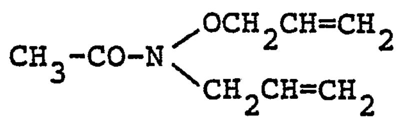, and

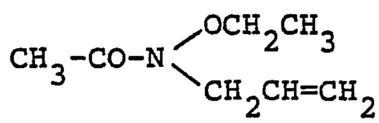.

5. The compound as claimed in claim 1 wherein $R^o$ is hydrogen and the compound is in the form of a salt with an acid.

6. The compound as claimed in claim 5 which is:

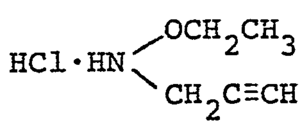.

7. The compound as claimed in any one of claims 1 to 3 wherein the compound is exclusive of compound of the formula

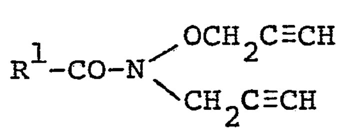

wherein $R^1$ is as defined hereinbefore.

8. The compound as claimed in any one of claims 1 to 3 wherein the compound is a compound of the formula

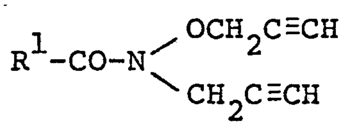

wherein $R^1$ is as defined hereinbefore.

9. An anthelmintic method which comprises administering orally to an animal afflicted with a parasite a compound represented by the formula

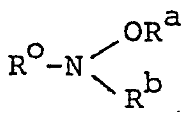 (A')

where: $R^o$ is a hydrogen atom or a group of the formula $R^1$-CO- where $R^1$ is a hydrogen atom or an alkyl or alkenyl having up to 5 carbon atoms, phenyl, or a substituted phenyl, the substituent being an alkyl having up to 3 carbon atoms, a halogen atom or nitro,

$R^a$ is an alkyl, alkenyl or alkynyl having up to 5 carbon atoms; and

$R^b$ is an alkenyl or alkynyl having up to 5 carbon atoms,

the combination of $R^o$, $R^a$ and $R^b$ being selected from the group consisting of:

(1) the combination in which

$R^o$ is $R^1$-CO-, $R^1$ being defined hereinabove,

$R^a$ is an alkyl or alkenyl having up to 5 carbon atoms, and

$R^b$ is $-CH_2C{\equiv}CH$;

(2) the combination in which

$R^o$ is $R^1$-CO-, $R^1$ being defined hereinabove,

$R^a$ is an alkyl having up to 5 carbon atoms, and

$R^b$ is $-CH_2CH=CH-R^3$, $R^3$ being hydrogen or methyl;

(3) the combination in which

$R^o$ is $R^1$-CO-, $R^1$ being defined hereinabove,

$R^a$ is $-CH_2CH=CH-R^3$,

$R^b$ is $-CH_2CH=CH-R^4$,

$R^3$ and $R^4$ being hydrogen or methyl, respectively;

(4) the combination in which

$R^o$ is H,

$R^a$ is as defined hereinabove, and

$R^b$ is as defined hereinabove; and

(5) the combination in which

$R^o$ is $R^1$-CO-, $R^1$ being defined hereinabove,

$R^a$ is $-CH_2C{\equiv}CH$, and

$R^b$ is $-CH_2C{\equiv}CH$.

10. The method as claimed in claim 9 wherein the compound is such that the substituent in $R^1$ is an alkyl having up to 3 carbon atoms or a halogen atom when $R^1$ is a substituted phenyl.

11. The method as claimed in claim 9 wherein the compound is such that $R^1$ is an alkyl having up to 5 carbon atoms.

12. The method as claimed in claim 11 selected from the group consisting of:

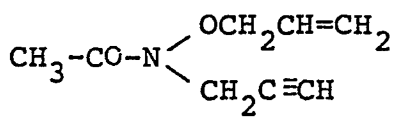
,

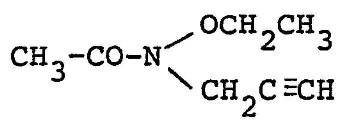
,

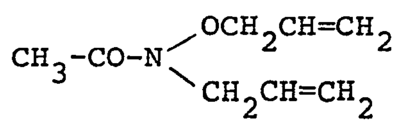
, and

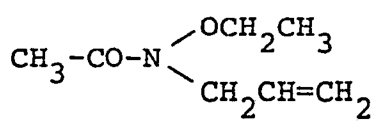
.

13. The method as claimed in claim 9 wherein the compound is such that $R^o$ is hydrogen, and the compound is in the form of a salt with an acid.

14. The method as claimed in claim 13 in which the compound is:

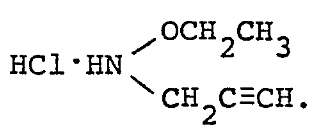

15. The method as claimed in any one of claims 9 to 11 wherein the compound is exclusive of a compound of the formula:

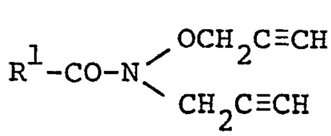

where $R^1$ is as defined hereinbefore.

16. The method as claimed in any one of claims 9 to 11 wherein the compound is a compound of the formula:

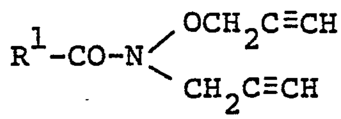

where $R^1$ is as defined hereinbefore.

17. An anthelmintic composition which comprises a compound represented by the following formula A' and a carrier:

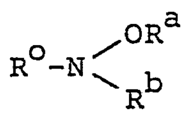 (A')

where: $R^o$ is a hydrogen atom or a group of the formula $R^1$-CO- where $R^1$ is hydrogen atom or an alkyl or alkenyl having up to 5 carbon atoms, phenyl, or a substituted phenyl, the substituent being an alkyl having up to 3 carbon atoms, a halogen atom or nitro,

$R^a$ is an alkyl, alkenyl or alkynyl having up to 5 carbon atoms; and

$R^b$ is an alkenyl or alkynyl having up to 5 carbon atoms,

the combination of $R^o$, $R^a$ and $R^b$ being selected from the group consisting of:

(1) the combination in which

$R^o$ is $R^1$-CO-, $R^1$ being defined hereinabove,

$R^a$ is an alkyl or alkenyl having up to 5 carbon atoms, and

$R^b$ is $-CH_2C\equiv CH$;

(2) the combination in which

$R^o$ is $R^1$-CO-, $R^1$ being defined hereinabove,

$R^a$ is an alkyl having up to 5 carbon atoms, and

$R^b$ is $-CH_2CH=CH-R^3$, $R^3$ being hydrogen or methyl;

(3) the combination in which

$R^o$ is $R^1$-CO-, $R^1$ being defined hereinabove,

$R^a$ is $-CH_2CH=CH-R^3$, and

$R^b$ is $-CH_2CH=CH-R^4$,

$R^3$ and $R^4$ being hydrogen or methyl, respectively;

(4) the combination in which

$R^o$ is H,

$R^a$ is as defined hereinabove, and

$R^b$ is as defined hereinabove; and

(5) the combination in which

$R^o$ is $R^1$-CO-, $R^1$ being defined hereinabove,

$R^a$ is $-CH_2C\equiv CH$, and

$R^b$ is $-CH_2C\equiv CH$.

18. The composition as claimed in claim 17, wherein the compound is such that the substituent in $R^1$ is an alkyl having up to 3 carbon atoms or a halogen atom when $R^1$ is a substituted phenyl.

19. The composition as claimed in claim 17 wherein the compound is such that $R^1$ is an alkyl having up to 5 carbon atoms.

20. The composition as claimed in claim 19 selected from the group consisting of:

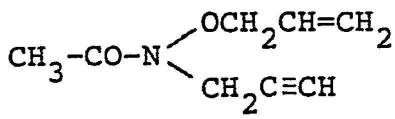
,

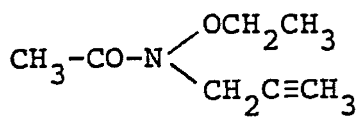
,

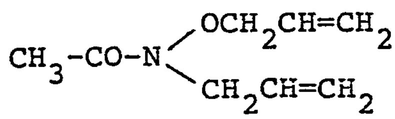
, and

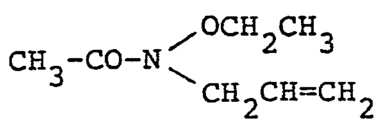
.

21. The composition as claimed in claim 17 wherein the compound is such that $R^o$ is hydrogen and the compound is in the form of a salt with an acid.

22. The composition as claimed in claim 21 wherein the compound is:

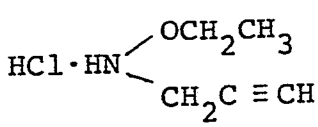
.

23. The composition as claimed in any one of claims 17 to 19 wherein the compound is exclusive of a compound of the formula:

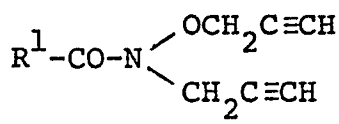

where $R^1$ is as defined hereinbefore.

24. The composition as claimed in any one of claims 17 to 19 wherein the compound is a compound of the formula:

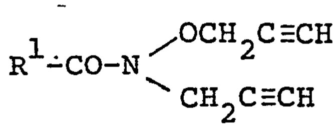

where $R^1$ is as defined hereinbefore.

25. A method of producing an N-propargyl-N-acylhydroxylamine derivative represented by the following formula (I-1) which comprises reacting a compound represented by the following formula (II-1) with a compound represented by the following formula (III-1) in the presence of a deacidification agent:

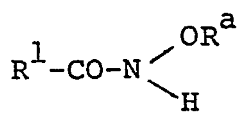 (II-1)

$CH\equiv CCH_2X$ (III-1)

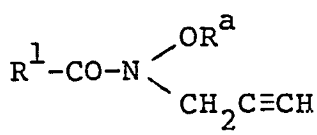 (I-1)

where: $R^1$ is a hydrogen atom or an alkyl or alkenyl having up to 5 carbon atoms, phenyl, or a substituted phenyl, the substituent being an alkyl having up to 3 carbon atoms, a halogen atom or nitro,

$R^a$ is an alkyl or alkenyl having up to 5 carbon atoms; and

X is a halogen atom.

26. A method of producing an N-alkenyl-N-acylhydroxylamine derivative which comprises reacting a compound represented by the following formula (II-2) with a compound represented by the following formula (III-2) in the presence of a deacidification agent:

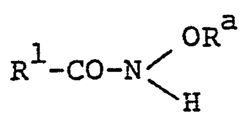

(II-2)

$R^3-CH=CHCH_2X$ (III-2)

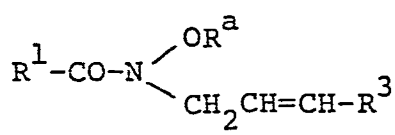

(I-2)

where: $R^1$ is a hydrogen atom or an alkyl or alkenyl having up to 5 carbon atoms, phenyl, or a substituted phenyl, the substituent being an alkyl having up to 3 carbon atoms; a halogen atom or nitro,

$R^a$ is an alkyl having up to 5 carbon atoms;

$R^3$ is a hydrogen atom or methyl; and

X is a halogen atom.

27. A method of producing a hydroxylamine derivative represented by the following formula (I-4) or its acid salt which comprises hydrolyzing a compound represented by the following formula (II-4) in the presence of an acid:

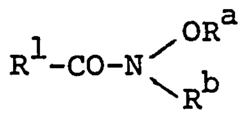

(II-4)

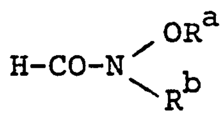

(I-4)

where: $R^1$ is a hydrogen atom or an alkyl or alkenyl having up to 5 carbon atoms, phenyl, or a substituted phenyl, the substituent being an alkyl having up to 3 carbon atoms;

$R^a$ is an alkyl or alkenyl having up to 5 carbon atoms; and

$R^b$ is $-CH_2C\equiv CH$.

28. A method of producing an N,O-dipropargyl-N-acylhydroxylamine derivative represented by the following formula (I-5) which comprises reacting a compound represented by the following formula (II-5) with a hydroxylamine source compound, and then with a compound represented by the following formula (III-5) in the presence of a deacidification agent:

$$R^1\text{-CO-OR}^c \qquad \text{(II-5)}$$

$$CH\equiv CCH_2Y \qquad \text{(III-5)}$$

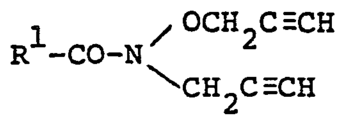

(I-5)

where: $R^1$ is a hydrogen atom or an alkyl or alkenyl having up to 5 carbon atoms, phenyl, or a substituted phenyl, the substituent being an alkyl having up to 3 carbon atoms, a halogen atom or nitro,

$R^c$ is an alkyl having up to 5 carbon atoms; and

Y is a halogen atom.

29. The method as claimed in claim 28 wherein the hydroxylamine source compound is selected from hydroxylamine and salts of hydroxylamine with an acid.

30. A method of producing an N,O-dipropargyl-N-acylhydroxylamine derivative represented by the following formula (I-5) which comprises reacting a hydroxamic acid or its salt with an alkali metal represented by the following formula (IV-5) with a compound represented by the following formula (III-5) in the presence of a deacidification agent:

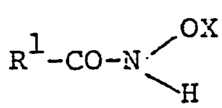

(IV-5)

$CH{\equiv}CCH_2Y$ (III-5)

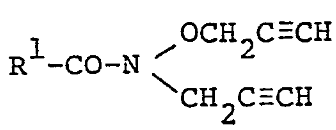

(I-5)

where: $R^1$ is a hydrogen atom or an alkyl or alkenyl having up to 5 carbon atoms, phenyl, or a substituted phenyl, the substituent being an alkyl having up to 3 carbon atoms, a halogen atom or nitro,

X is a hydrogen atom or an alkali metal; and

Y is a halogen atom.

31. A method of producing an N,O-dipropargyl-N-acylhydroxylamine derivative represented by the following formula (I-5) which comprises reacting a compound represented by the following formula (V-5) with a compound represented by the following formula (III-5) in the presence of a deacidification agent:

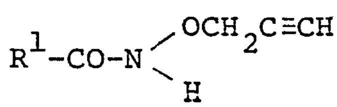

(V-5)

$CH\equiv CCH_2Y$ (III-5)

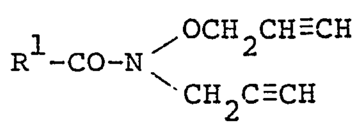

(I-5)

where: $R^1$ is a hydrogen atom or an alkyl or alkenyl having up to 5 carbon atoms, phenyl, or a substituted phenyl, the substituent being an alkyl having up to 3 carbon atoms, a halogen atom or nitro; and

Y is a halogen atom.